# EUROPEAN PATENT SPECIFICATION

(11) **EP 1 189 631 B1**
(45) Date of publication and mention of the grant of the patent: **01.12.2004**
(21) Application number: 00907858.5
(22) Date of filing: 09.03.2000
(51) Int. Cl.: A61K 38/45, A61K 48/00, A61K 39/395, C12N 9/12, C12N 15/54, A01K 67/027, C07K 16/40, C12Q 1/48, A61P 25/28

(54) **NEURODEGENERATIVE DISORDER RELATED GENE**
NEURODEGENERATIVE-ERKRANKUNG VERWANDTES GEN
GENE APPARENTE A UN TROUBLE NEURO-DEGENERATIF

(30) Priority: 09.03.1999 GB 9905218
(43) Date of publication of application: 27.03.2002
(73) Proprietor: THE UNIVERSITY COURT OF THE UNIVERSITY OF GLASGOW, Glasgow G12 8QQ (GB)
(72) Inventor: DAVIES, Roger, Wayne, Glasgow G11 6NU (GB); PAYNE, Anthony, Philip, University of Glasgow, Glasgow G12 8QQ (GB); SUTCLIFFE, Roger, Graham, Glasgow G11 6NU (GB)
(74) Representative: Forrest, Graham Robert
(86) International application number: PCT/GB2000/000860
(87) International publication number: WO 2000/053218

(56) References cited:
- WO-A-95/02069
- WO-A-98/39444
- ABELOVICH, A. ET AL: "Modified hippocampal long-term potentiation in PKCgamma-mutant mice" CELL, vol. 75, 31 December 1993 (1993-12-31), pages 1253-1262, XP000910293
- DATABASE WPI Section Ch, Week 199444 Derwent Publications Ltd., London, GB; Class B05, AN 1994-354659 XP002140960 & JP 06 279311 A (NIPPON SHOJI KK), 4 October 1994 (1994-10-04)
- KOLB, H. ET AL: "Differential staining of neurons in the human retina with antibodies to protein kinase C isozymes" VISUAL NEUROSCIENCE, vol. 10, 1993, pages 341-351, XP000915830
- CAZAUBON, S. ET AL: "Effector dependant conformational changes in protein kinase C-gamma through epitope mapping with inhibitory monoclonal antibodies" EUROPEAN JOURNAL OF BIOCHEMISTRY, vol. 194, 1990, page 799-804 XP002109461
- CAZAUBON, S. ET AL: "Monoclonal antibodies to protein kinase C-gamma: functional relatonship between epitopes and co-factor binding sites" EUROPEAN JOURNAL OF BIOCHEMISTRY, vol. 182, 1989, pages 401-406, XP000915810
- SMALLWOOD, J. I. ET AL: "An apparently novel protein of human leukocytes, reactive with an antibody to protein kinase C-gamma, is rapidly modified upon cell activation: Initial characterization in neutrophils and their cytoplasts" INFLAMMATION, vol. 22, no. 1, February 1998 (1998-02), pages 1-28, XP000915851
- FAVIT, A. ET AL: "Alzheimer's-specific effects of soluble beta-amyloid on protein kinase-C-alpha and gamma degradation in human fibroblasts" PROC. NAT. ACAD. SCI. USA, vol. 95, 12 May 1998 (1998-05-12), pages 5562-5567, XP000901213
- SHIMOHAMA, SHUN ET AL: "Signal transduction mechanisms in Alzheimer disease" ALZHEIMER DISEASE AND ASSOCIATED DISORDERS, vol. 9 (SUPP 2), 1995, pages 15-22, XP000915805
- CRAIG, N. J. ET AL: "Genetic and physical mapping of the agu mutation." SOCIETY FOR NEUROSCIENCE ABSTRACTS, (1997) VOL. 23, NO. 1-2, PP. 1873. MEETING INFO.: 27TH ANNUAL MEETING OF THE SOCIETY FOR NEUROSCIENCE NEW ORLEANS, LOUISIANA, USA OCTOBER 25-30, 1997 , XP000915938

## Description

The present invention relates to the use of a polynucleotide fragment encoding protein kinase C type I (PKC type I) as well as fragments thereof, mutant polynucleotide fragments of the polynucleotide fragment, a recombinant vector comprising such a polynucleotide fragment or mutant polynucleotide fragment, a host cell comprising said polynucleotide fragment or mutant polynucleotide fragment, a host cell comprising a recombinant vector comprising said polynucleotide fragment or mutant polynucleotide fragment, a recombinant or synthetic polypeptide thereto, antibodies specific to said polypeptide, antisense oligonucleotides complementary to said polynucleotide fragment or mutant polynucleotide fragment, pharmaceutical compositions comprising said recombinant or synthetic polypeptide, pharmaceutical compositions comprising said antisense oligonucleotides and pharmaceutical compositions comprising said polynucleotide fragment for use in prophylaxis and/or as a therapeutic agent in animals, particularly humans, as well as uses of said polynucleotide fragment or mutant polynucleotide fragment, antisense oligonucleotides, antibodies and/or polypeptides in diagnostic and/or screening assays.

Degenerative disorders of the nervous system, such as Parkinson's Disease, Alzheimer's Disease and Huntington's Disease, have provided a challenge for many years, in both the basic research and clinical contexts. A major problem has been the lack of animal models which accurately mimic the clinical conditions since a large proportion of research is carried out initially on animals in which a disorder has been created by experimental manipulation of the central nervous system (CNS). Examples of experimental manipulation of the CNS in the field of Parkinson's Disease include rodent studies which have relied on lesioning the nigrostriatal system with, for example, the toxin 6-hydroxydopamine. Several genetic mouse models of movement disorders exist, although the majority of such mutants breed poorly and have a reduced life expectancy which limits their efficacy for study of the long term progression of the various conditions. One such model, the weaver mouse, has a deficiency in its dopaminergic systems and has thus been proposed as a model of Parkinson's disease. However, this mutant also possesses severe cerebellar abnormalities and the resulting behaviours may mask those generated by the Parkinsonian-like dopamine deficiencies.

A mutant rat strain (AS/AGU) which had risen spontaneously in a closed breeding colony of Albino-Swiss (AS) rats at the Department of Anatomy, Glasgow University (AGU) was initially described by Clarke & Payne (1994) European Journal of Neuroscience 6 pp885 - 888. The mutant rat displayed a movement disorder which consisted primarily of a difficulty in initiating movement, with a staggering gait and hind limb rigidity. The animals were in good general health and were fertile. Successful breeding between affected individuals resulted after several generations in all off-spring bearing the same motor deficits. Subsequent genetic analysis has shown that the mutation is an autosomal recessive. The gait disturbances are first detected at around postnatal day 10 and become progressively more severe. The life expectancy of these animals is around 18 months, somewhat foreshortened when compared with the parent Albino-Swiss strain, whose life expectancy is more than 2 years.

A 60% deficit in dopaminergic cell bodies in the substantia nigra pars compacta was detected in the AS/AGU mutants compared to the AS controls at 12 months of age. This provided evidence for basal ganglia involvement and suggested that the disorder could be pathologically very similar to human Parkinson's Disease, which is characterised by the loss of dopaminergic neurons in the substantia nigra pars compacta (SNpc).

Further research using the micropunch procedure revealed depletions of tissue (combined pre-synaptic and released) dopamine in the dorsal and lateral striatum of 30% and 20% respectively in 12 month old AS/AGU mutants compared to age matched controls (Campbell et al 1996 Neuroscience Letters, 213 pp 173 - 176). This was an expected consequence of loss or decreased function of dopaminergic neurons in the SNpc, which project to the striatum.

When an age range study was carried out on rats of 3 months, 6 months, 9 months and 12 months old, it was found that tissue dopamine depletion in the dorsal and lateral striatum of AS/AGU mutants increased with age from 6 months onwards, thereby demonstrating that the disorder was progressive (Campbell et al, 1997 Neuroscience Letters 239 pp54 - 56).

Extracellular levels of dopamine and its metabolite, 3,4-dihydroxyphenylacetic acid (DOPAC) were measured by microdialysis in the corpus striatum of conscious AS/AGU mutant rats. Extracellular levels of dopamine were found to be very significantly reduced-approximately 80% in 9 month old AS/AGU rats compared to age matched AS controls. This was also found to be progressive over an age range. The extracellular levels of the degradation product of dopamine, ie. DOPAC, was found to be elevated in AS/AGU rats compared to AS controls at all ages (Campbell et al, 1998 Neuroscience 85 pp323 - 325).

Local cerebral glucose utilisation is a measure of the metabolic activity of cells in various brain regions. This was measured in AS/AGU rats and statistically significant decreases in glucose utilisation were apparent in 12 out of 44 brain regions examined in 12 month rats. The most significant decreases were found in the substantia nigra pars compacta and the medidical geniculate. Lesser effects were observed in the subthalamic nucleus in extra pyramidal regions and several limbic structures. The cerebellum and white matter areas were not affected. This evidence suggests that the dopaminergic cells of the SNpc are in some way metabolically comprised (Lam et al, 1998 European Journal of Neuroscience 10 pp1963 - 1967).

When L-Dopa was administered to AS/AGU rats it was shown to greatly enhance the ability of the AS/AGU rats to perform a number of locomotor tasks such as mid-air righting and walking down an inclined ramp. This was also observed when foetal midbrain cells were transplanted into the striatum. L-Dopa treatment and foetal midbrain transplants are known to improve the symptomatic state of human Parkinson's Disease patients. This result revealed that the majority of the movement disorder, and thus the neurodegenerative damage in the AS/AGU is due to loss of dopaminergic neuron function in the SNpc (Payne et al, 1998 Movement Disorders 13 pp832 - 834).

All this work suggested that the AS/AGU rat may be a good candidate as a phenotypic model for Parkinson's Disease. However, there was no evidence of how the AS/AGU rat may be affected at the genetic level.

The PKC gamma gene encoding the protein kinase C type I (PKC type I) isoenzyme has previously been studied in mice and transgenic mice lacking the type I subtype have been produced (Abeliovich et al 1993, Cell, 75, pp1253 - 1262). The null mutant mice produced displayed little or no behavioural impairment.

A mutation in the PKCγ gene in humans has been shown to be associated with the disorder retinitis pigmentosa (RP) see Al-Maghtheh et al, 1998 Am. J. Hum. Genetics 62 pp1248 - 1252. However, there was no suggestion that the mutation was associated with any additional neurological disorder such as Parkinson's Disease, Alzheimer's Disease or Huntington's Disease.

The present invention is based on the discovery by the present inventors that a mutation(s) within the PKCγ gene encoding the type I subtype of protein kinase C is associated with the AS/AGU mutant rat.

Thus, in a first aspect, the present invention provides use of a polynucleotide fragment comprising the PKCγ gene encoding the type I subtype of protein kinase C in the manufacture of a medicament for treating a neurodegenerative disorder.

In a further aspect, the present invention provides use of a polypeptide which comprises protein kinase C type I in the manufacture of a medicament for treating a neurodegenerative disorder.

Typically the medicament may be used to treat mammals, in particular humans. The neurodegenerative disorder may be a degenerative disorder of the central nervous system, such as Alzheimer's Disease, or more particularly, a neurodegenerative disorder associated with dopaminergic cell degeneration and/or movement impairments such as Parkinson's Disease, or Huntington's Disease/Chorea, Dementia with Lewy bodies, Multiple-system atrophy (including striatonigral degeneration, sporadic olivopontocerebellar atrophy and shy-drager syndrome), Progressive supranuclear palsy, cortical-basal ganglionic (corticobasal) degeneration, vascular Parkinsonism or ballism.

"Polynucleotide fragment" as used herein refers to a polymeric form of nucleotides of any length, both to ribonucleic acid sequences and to deoxyribonucleic acid sequences. In principle, this term refers to the primary structure of the molecule, thus this term includes double stranded and single stranded DNA, as well as double and single stranded RNA, and modifications thereof.

In general, the term "polypeptide" refers to a molecular chain of amino acids with a biological activity. It does not refer to a specific length of the product, and if required it can be modified *in vivo* and/or *in vitro,* for example by glycosylation, myristoylation, amidation, carboxylation or phosphorylation; thus inter alia peptides, oligopeptides and proteins are included. The polypeptides disclosed herein may be obtained, for example, by synthetic or recombinant techniques known in the art.

Thus the term extends to cover, for example, polypeptides obtainable from various transcripts and splice variants of these transcripts from the PKCγ gene. Additionally, functional domains may be observed in the protein and isolated polypeptides relating to these functional domains may be of particular use. For example, a regulatory domain, a kinase domain and an ATP-binding domain have been observed in the PKC type I polypeptide. The present invention also relates to polynucleotide fragments comprising a nucleotide sequence encoding such functional domain polypeptides.

It will be understood that for the PKCγ nucleotide and polypeptide sequences referred to herein, natural variations can exist between individuals. These variations may be demonstrated by amino acid differences in the overall sequence or by deletions, substitutions, insertions or inversions of amino acids in said sequence. All such variations are included in the scope of the present invention.

As is well known in the art, the degeneracy of the genetic code permits substitution of bases in a codon resulting in a different codon encoding the same amino acid. Consequently, it is clear that any such derivative nucleotide sequence based on the sequences disclosed herein is also included in the scope of the present invention.

Thus, the present invention also includes nucleotide sequences similar to the polynucleotide sequences disclosed herein. It is understood that similar sequences include sequences which remain hybridised to the polynucleotide sequences of the present invention under stringent conditions. Typically, a similar test sequence and a polynucleotide sequence of the present invention are allowed to hybridise for a specified period of time generally at a temperature of between 50 and 70°C in double strength SSC (2 x NaCl 17.5g/l and sodium citrate (SC) at 8.8 g/l) buffered saline containing 0.1% sodium dodecyl sulphate (SDS) followed by rinsing of the support at the same temperature but with a buffer having a reduced SSC concentration. Depending upon the degree of similarity of the sequences, such reduced concentration buffers are typically single strength SSC containing 0.1% SDS, half strength SSC containing 0.1% SDS and one tenth strength SSC containing 0.1% SDS. Sequences having the greatest degree of similarity are those the hybridisation of which is least affected by washing in buffers of reduced concentration. it is most preferred that the similar and inventive sequences are so similar that the hybridisation between them is substantially unaffected by washing or incubation in one tenth strength SSC containing 0.1% SDS.

Furthermore, fragments derived from the PKCγ gene or PKC type I protein which still display PKCγ specific properties or PKC type I specific properties are also included in the present invention. "PKCγ specific properties" is understood to relate to biological functions which are attributable to naturally-occurring PKCγ gene and "PKC type I specific properties" is understood to relate to biological functions which are attributable to naturally-occurring PKC type I protein. This may include fusion proteins.

All such modifications mentioned above resulting in such derivatives of PKCγ are covered by the present invention so long as the characteristic PKCγ properties remain substantially unaffected in essence.

The present inventors applied genetic mapping techniques in order to ascertain the genotypic variation displayed in the AS/AGU rat using the process of "positional cloning" (Collins, 1992, Nature Genetics, 1, 3 - 6). This mapping revealed that the AS/AGU mutation was in close proximity to the genetic marker R158 (Serikawa et al, 1992, Genetics 137, pp701 - 721). Nucleotide sequencing was then carried out, which upon comparison with wild type AS sequence, revealed a mutation in the PKCγ gene.

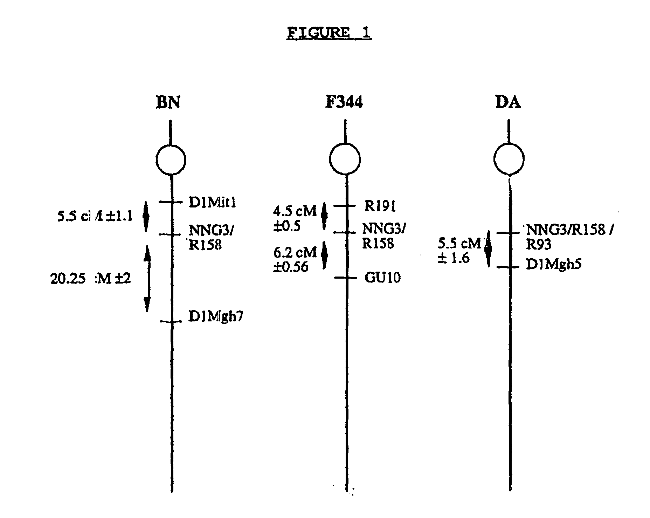

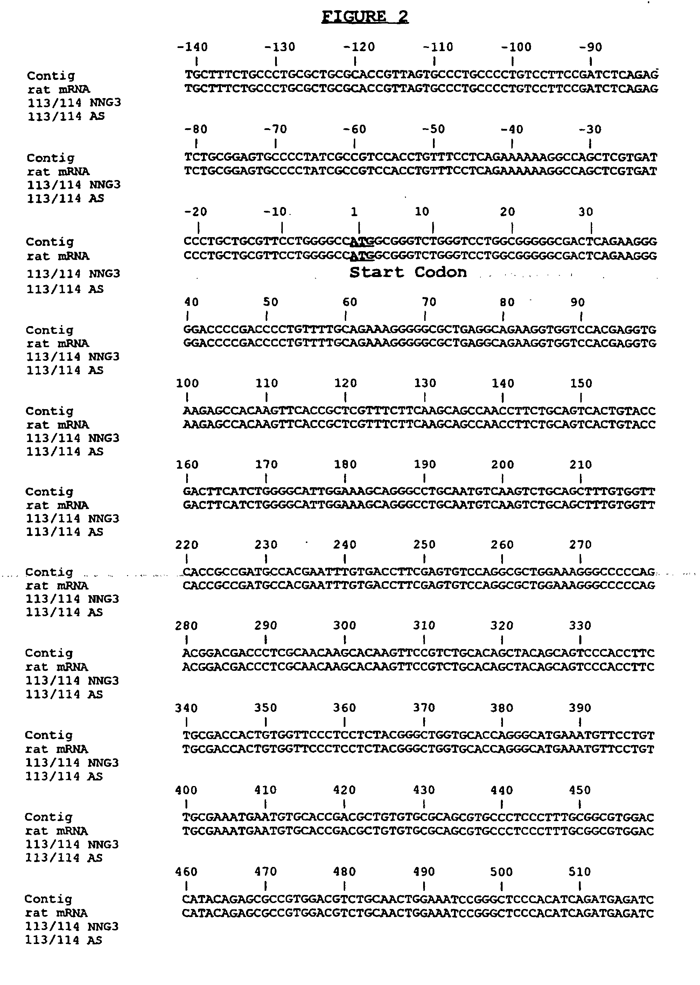

A point mutation was observed at nucleotide 841 of the rat PKCγ messenger RNA sequence(shown in Figure 1 where nucleotide numbering is such that base A of the ATG start codon is no. 1) such that a guanine base, present in the AS gene sequence, was mutated to a thymine base in the AS/AGU mutant sequence. This transversion mutation results in the generation of an in-frame stop codon which upon translation of the PKCγ gene would result in a prematurely terminated protein the length of which would be 280 amino acids. It is postulated that this truncated protein would not possess several of the domains present in the wild-type protein. That is, the regulatory domain would be present in the truncated protein and not the kinase or ATP-binding domains.

Since this genotypic mutation is associated with the phenotypic movement disorder observed in the AS/AGU mutant rat, observation of such a mutation in PKCγ may be used in a predictive test for neurodegenerative disorders, such as Parkinson's disease, Alzheimer's Disease or Huntington's Disease. Alternatively, measuring levels of PKC type I protein levels and/or activity may be useful in a predictive or diagnostic test for neurodegenerative disorders.

Thus, in a further aspect, the present invention provides a method of testing an animal thought to have or be predisposed to having a neurodegenerative disorder which comprises detecting the presence of a mutation in the PKCγ gene and/or its associated promoter.

Typically, the mutation(s) may result in a truncated product from the PKCγ gene being produced. More particularly the mutation may occur in the 5' half of the gene. For example the mutation may be a point mutation such as at position 841 of the rat PKCγ gene or similar region of the PKCγ gene from another species. The skilled man will immediately appreciate that the information presented herein relating to the rat PKCγ may easily be equated or correlated with a similar mutation at a corresponding location in the PKCγ gene from another species, such as humans. Thus, the present invention provides the means with which to test humans for a similar mutation in the human PKCγ gene and therefore predict if the test subject has or is predisposed to developing a neurodegenerative disorder for example, Parkinson's Disease, Alzheimer's Disease or Huntington's Disease.

Typical techniques for detecting the mutation may include restriction fragment length polymorphism, hybridisation techniques, DNA sequencing, exonuclease resistance, microsequencing, solid phase extension using ddNTPs, extension in solution using ddNTPs, oligonucleotide ligation assays, methods for detecting single nucleotide polymorphisms such as dynamic allele-specific hybridisation, ligation chain reaction, mini-sequencing, DNA "chips", allele-specific oligonucleotide hybridisation with single or dual-labelled probes merged with PCR or with molecular beacons, and others.

Altered levels of the mRNA transcripts encoding the truncated PKC type I polypeptide have been observed. Thus, detection of altered levels of the mRNA transcript or mRNA precursors, such as nascent RNA, may be used to diagnose if the test subject has or is predisposed to developing a neurodegenerative disorder.

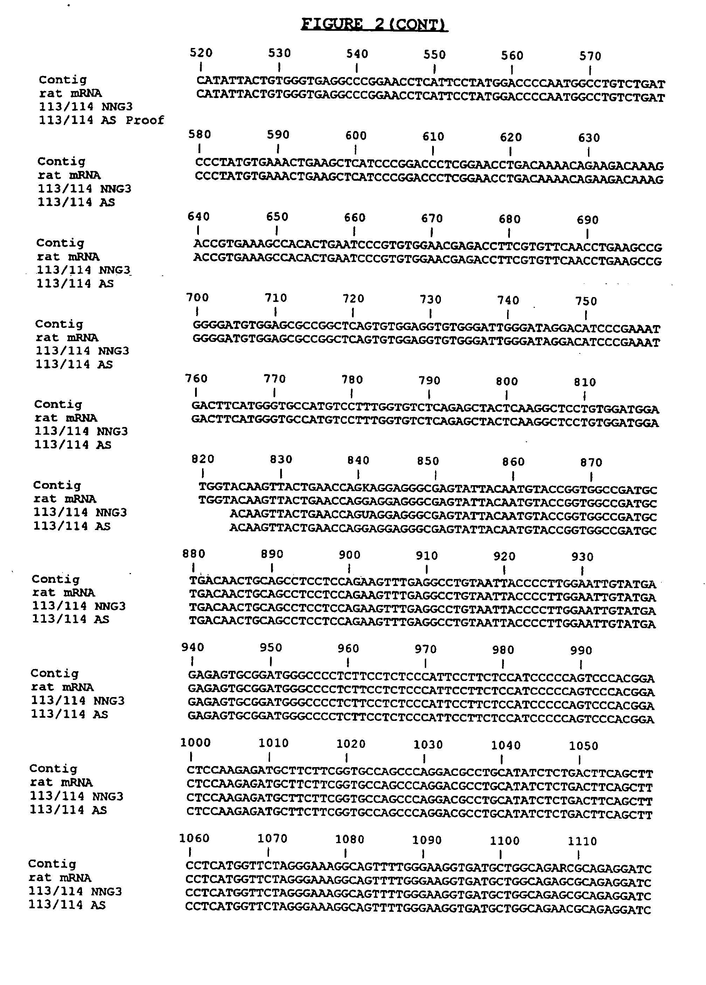

The information presented herein may also be used to genetically manipulate the wild-type PKCγ gene, mutant PKCγ gene or derivatives thereof, for example to clone the gene by recombinant DNA techniques generally known in the art. Cloning of homologous genes from other species of mammal may be performed with this information by widely known techniques; for example, suitable primers may be designed to a consensus region and/or functional domains of the sequence shown in Figure 2 and such primers used as probes for cloning homologous genes from other organisms.

Moreover, mammalian PKCγ mutant and wild-type nucleotide sequences of the present invention are preferably linked to expression control sequences. Such control sequences may comprise promoters, operators, inducers, ribosome binding sites etc. Suitable control sequences for a given host may be selected by those of ordinary skill in the art.

A nucleotide sequence according to the present invention can be ligated to various expression-controlling DNA sequences, resulting in a so-called recombinant nucleic acid molecule. Thus, the present invention also includes an expression vector comprising an expressible PKCγ mutant or wild-type nucleotide sequence. Said recombinant nucleic acid molecule can then be used for transformation of a suitable host.

Such recombinant nucleic acid molecules are preferably derived from for example, plasmids, or from nucleic acid sequences present in bacteriophages or viruses and are termed vector molecules.

Specific vectors which can be used to clone nucleotide sequences according to the invention are known in the art (eg. Rodriguez and Denhardt, editors, Vectors: A survey of molecular cloning vectors and their uses, Butterworths, 1988).

The methods to be used for the construction of a recombinant nucleic acid molecule according to the invention are known to those of ordinary skill in the art and are *inter alia* set forth in Sambrook et al, Molecular Cloning: A Laboratory Manual, Cold Spring Harbour Laboratory, 1989.

The present invention also relates to a transformed cell comprising the mutant or wild-type nucleic acid molecule in an expressible form. "Transformation", as used herein, refers to the introduction of a heterologous nucleic acid sequence into a host cell *in vivo*, *ex vivo* or *in vitro* irrespective of the method used, for example, by calcium phosphate co-precipitation, direct uptake or transduction.

The heterologous nucleic acid sequence may be maintained through autonomous replication or alternatively may be integrated into the host's genome. The recombinant DNA molecules are preferably provided with appropriate control sequences compatible with the designated host which can regulate the expression of the inserted nucleic acid sequence.

The most widely used hosts for expression of recombinant nucleic acid molecules are bacteria, yeast, insect cells and mammalian cells. Each system has advantages and disadvantages in terms of the vector used, potential ease of production and purification of a recombinant polypeptide and authenticity of product in terms of tertiary structure, glycosylation state, biological activity and stability and will be a matter of choice for the skilled addressee.

In addition to promoting expression of a PKC type I polypeptide in cells, in certain circumstances it may be advantageous to substantially prevent or reduce the expression or activity of the native PKC type I in a host, for example, for the production of animal models for use in drug screening, or particularly if the native PKC type I is of a mutant form.

Thus, according to a further aspect of the invention, there is provided an antisense nucleotide fragment complementary to a PKCγ nucleotide sequence of the present invention. Included in the scope of "antisense nucleotide fragment" is the use of synthetic oligonucleotide sequences, or of equivalent chemical entities known to those skilled in the art, for example, peptide nucleic acids. Further, such sequences can be used as part of ribozyme and/or triple helix sequences, which may also be useful for target gene regulation. Also provided is a nucleotide fragment comprising a nucleotide sequence which, when transcribed by the cell, produces such an antisense fragment. Typically, antisense RNA fragments will be provided which bind to complementary PKCγ mRNA fragments to form RNA double helices, allowing RNAse H to cleave the molecule and rendering it incapable of being translated by the cell into polypeptides.

A further aspect of the present invention provides antibodies specific to the PKC type I polypeptide or truncated polypeptide as identified herein or epitopes thereof. Production and purification of antibodies specific to an antigen is a matter of ordinary skill, and the methods to be used are clear to those skilled in the art. The term antibodies can include, but is not limited to, polyclonal antibodies, monoclonal antibodies (mAbs), humanised or chimeric antibodies, single chain antibodies, Fab fragments, F(ab')₂ fragments, fragments produced by a Fab expression library, anti-idiotypic (anti-Id) antibodies, and epitope binding fragments of any of the above. Such antibodies may be used in modulating the expression or activity of the full length or truncated PKC type I polypeptide, or in detecting said polypeptide *in vivo* or *in vitro.*

The present invention further provides a recombinant or synthetic PKC type I polypeptide for the manufacture of reagents for use as prophylactic or therapeutic agents in mammals. In particular, the invention provides pharmaceutical compositions comprising the recombinant or synthetic PKC type I polypeptide together with a pharmaceutically acceptable carrier therefor.

According to a still further aspect of the present invention, there is provided use of a polypeptide or nucleic acid sequence as hereinbefore described for promoting nervous system degeneration for use in, for example, production of animal models which may be used in drug screening.

There is also provided use of a polypeptide or nucleic acid sequence as hereinbefore described in preventing, delaying, treating or inhibiting degeneration of the nervous system. There is further provided a method of preventing, delaying, treating or inhibiting degeneration of the nervous system comprising providing PKC type I polypeptide to a subject displaying or predicted to display degeneration of the nervous system. Such a method may find particular application in the treatment of degenerative disorders of the central nervous system, such as Alzheimer's Disease, or more particularly neurodegenerative disorders associated with movement impairment such as Parkinson's Disease or Huntington's Chorea.

Also provided is a method of preventing, delaying, treating or inhibiting degeneration of the nervous system comprising providing a subject with a nucleotide sequence or an antibody which substantially prevents or reduces expression or activity of a mutant PKC type I polypeptide.

A yet further aspect of the present invention provides use of polypeptides or nucleic acid sequences as hereinbefore described in the treatment of degenerative disorders of the nervous system, such as Parkinson's Disease, Alzheimer's Disease or Huntington's Disease. One such envisaged treatment may be by way of so-called gene therapy in which a wild-type PKCγ gene is introduced to a subject possessing a mutant PKCγ gene in order to counter the effects of the mutant PKCγ gene. This may be performed by the implantation of cells, such as fibroblasts expressing human or mammalian PKCγ fused to herpes virus VP22 protein that will transfer itself and PKCγ into adjacent neurons. Transformation of the cells to be implanted may be performed *in vitro* by any number of techniques, including physical means such as microinjection, electroporation, bioballistic or particle bombardment, jet injection or others; by chemical means such as using calcium phosphate, DEAE dextran, polylysine conjugates, "starburst" dendrimer conjugates, polybrene-dimethyl sulphoxide. The PKCγ gene itself, within an appropriate vector end-linked to an appropriate expression system, may be directly delivered via receptor-mediated uptake systems such as asialoglycoprotein and transferrin, liposomes, virus-like particles, intracellular targeting ligands and others; and by biological means including retroviral vectors such as Moloney murine leukaemia virus, adenovirus vectors and adeno-associated virus vectors, Herpes Simplex virus vectors, Semliki Forest virus vectors, Sindbis virus vectors and others.

The present invention also relates to methods for prognostic and diagnostic evaluation of various degenerative disorders of the nervous system, and for the identification of subjects who are predisposed to such disorders, for example determination of allelic variation by determination of the PKCγ nucleotide sequence in an individual and/or detection of truncated transcripts derived from the PKCγ gene, whether they are mRNA or polypeptide or measurements of PKC type I levels and/or activity. Furthermore, the invention provides methods for evaluating the efficacy of drugs for such disorders and monitoring the progress of patients involved in clinical trials for the treatment of such disorders.

The invention further provides methods for the identification of compounds which modulate the expression of a mutated or wild-type PKCγ gene and/or the activity of the product(s) of such a mutant or wild-type PKCγ gene which may be involved in processes relevant to degenerative disorders of the nervous system. Such compounds may include agonists, defined as compounds which increase the expression of a mutated or wild-type PKCγ gene and/or activity of the product(s) of such a mutant or wild-type PKCγ gene, and/or antagonists, defined as compounds which decrease the expression of a mutated or wild-type PKCγ gene and/or the activity of the product(s) of such a mutant or wild-type PKCγ gene. Thus, the present invention in a further aspect also provides agonists and/or antagonists.

The biological function of the PKCγ gene can be more directly assessed by utilizing relevant *in vivo* and *in vitro* systems. *In vivo* systems can include, but are not limited to, animal systems which naturally exhibit the symptoms of nervous system disorders, or ones which have been engineered to exhibit such symptoms. Further, such systems can include, but are not limited to, transgenic animal systems. *In vitro* systems can include, but are not limited to, cell-based systems comprising PKCγ gene/PKC type I protein expressing cell types. The cells can be wild type cells, or can be non-wild type cells containing modifications known or suspected of contributing to the disorder of interest.

In further characterising the biological function of the PKCγ mutant or wild-type gene, the expression of the PKCγ mutant or wild-type gene can be modulated within the *in vivo* and/or *in vitro* systems, i.e. either overexpressed or underexpressed in, for example, transgenic animals and/or cell lines, and its subsequent effect on the system can then be assayed. Alternatively, the activity of the product of the identified gene can be modulated by either increasing or decreasing the level of activity in the *in vivo* and/or *in vitro* system of interest, and its subsequent effect then assayed.

The information obtained through such characterisations can suggest relevant methods for the treatment or control of nervous system disorders. For example, relevant treatment can include a modulation of gene expression and/or gene product activity. Characterisation procedures such as those described herein can indicate whether such modulation should be positive or negative. As used herein, "positive modulation" refers to an increase in gene expression or activity of the gene or gene product of interest. "Negative modulation", as used herein, refers to a decrease in gene expression or activity.

*In vitro* systems can be designed to identify compounds capable of binding the PKCγ mutant or wild-type gene products of the invention. Compounds identified, for example, could be useful in modulating the activity of wild type or mutant PKCγ gene products, could be useful in elaborating the biological function of the PKCγ gene products, or could disrupt or enhance normal PKCγ gene product interactions, for example, the activators or inhibitors of PKC type I protein as disclosed in Keenan et al, 1997, FEBS Letters, 415 pp101 - 108. Such compounds may be investigated for their use in treating or alleviating motor impairment and/or dopaminergic cell degeneration disorders.

These and other aspects of the invention shall now be further described, by way of example only, and with reference to the accompany figures which show:
**Figure 1** is a diagram illustrating the region of rat genome selected to contain recombination events and genetic markers used to establish panels of backcross progeny recombinant in the interval containing the mutant PKCγ gene hereinafter referred to as nng3. The three backcrosses (BN x NNG3) F1 x NNG3, (F344 x NNG3) F1 x NNG3 and (DA x NNG3) F1 x NNG3 are labelled BN, F344 and DA respectively;
**Figure 2** is a sequence alignment of sequence obtained from the PKCγ gene in rat strains NNG3 and AS. Sequences are aligned to the rat (Rattus rattus) mRNA sequence obtained from NCBI (Accession number: X07287). The point mutation identified within the nng3 sequence is shown in bold and underlined at nucleotide 841. The translation start site is shown underlined, and the microsatellite defining the marker R158 (within the 3'UTR) is shown in italics. The primers defining the marker R158 are shown underlined and indicated by arrows. The normal translation stop site is shown at nucleotide 2093, in bold and underlined;

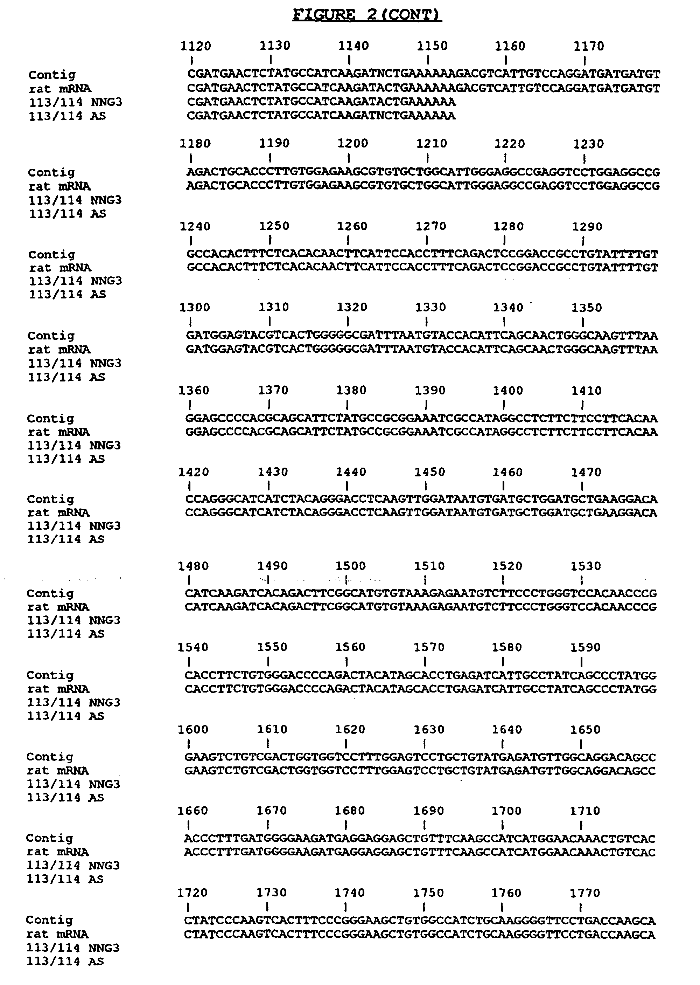

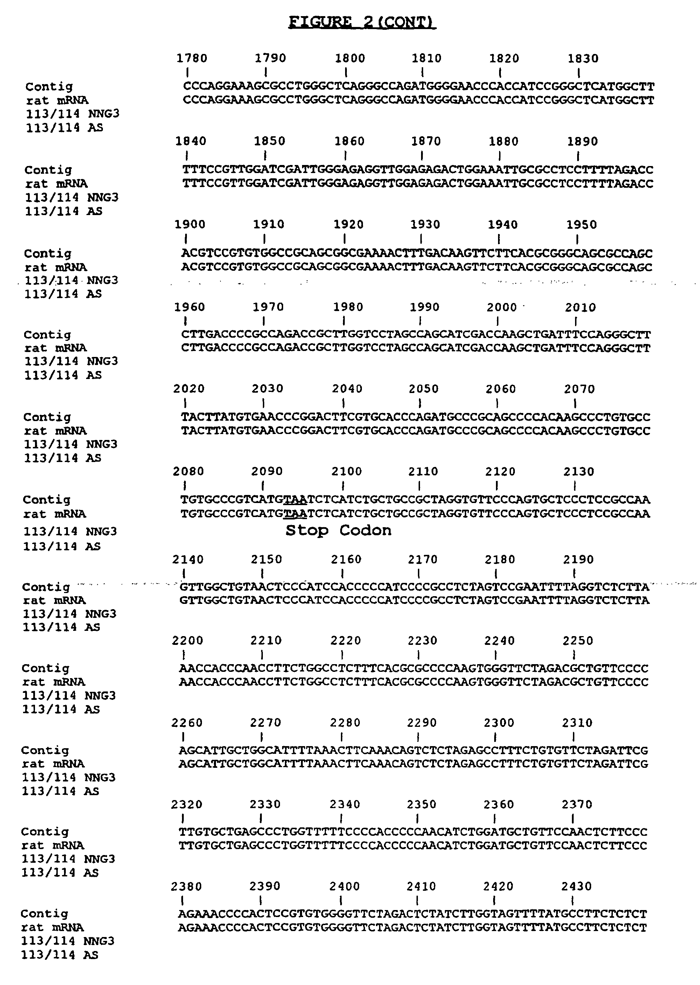

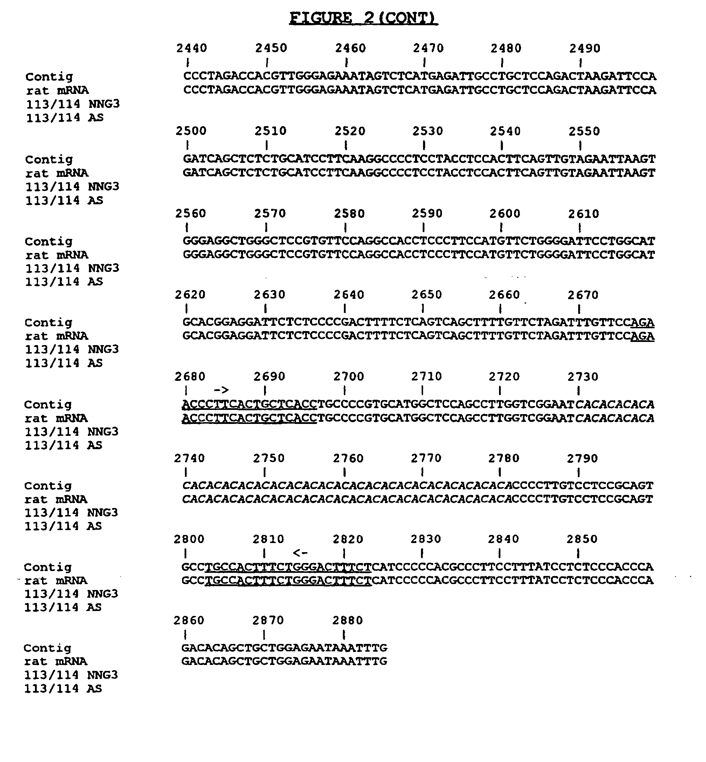

**Figure 3** is an alignment of PKCγ DNA sequence as illustrated in Figure 2 with the predicted amino acid sequence. The sequence illustrated is from the rat strain *Rattus rattus,* and the nucleotide conversion seen in the sequence illustrated in Figure 2 is given in bold, below the sequence for amino acid number 281. The normal translational start and stop codons are shown in bold. The site at which a codon encoding the amino acid Glu is changed by the mutation to a STOP codon, resulting in a polypeptide terminator, is also indicated and is shown in bold;

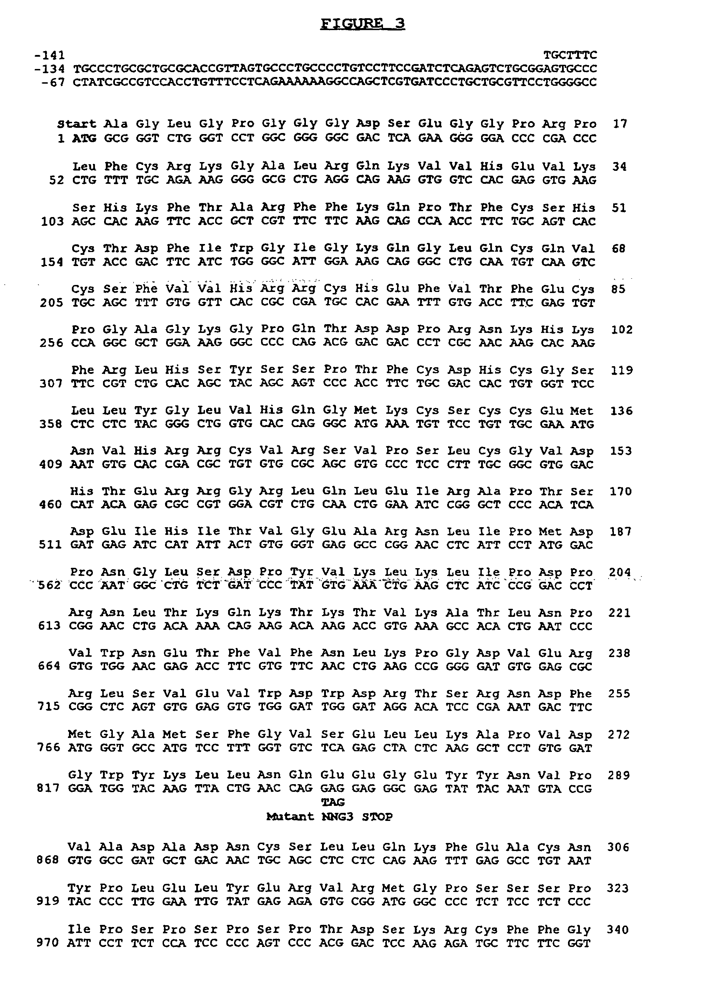

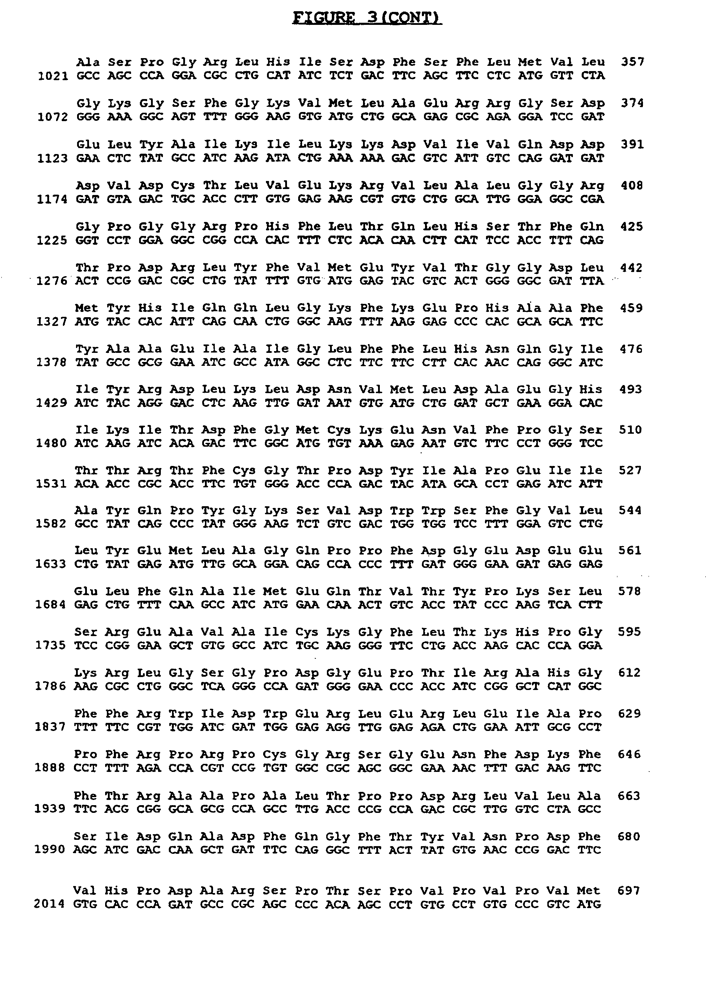

**Figures 4 (a) and (b)** are immunocytochemistry stainings of rat brains with anti-PKCγ antibody. Figure 4(a) illustrates AS control rat brain stained with PKCγ, with the Purkinje cell layers 10 and granule cell layers 15 indicated. Figure 4(b) illustrates NNG3 rat brain stained with anti-PKCγ antibody;

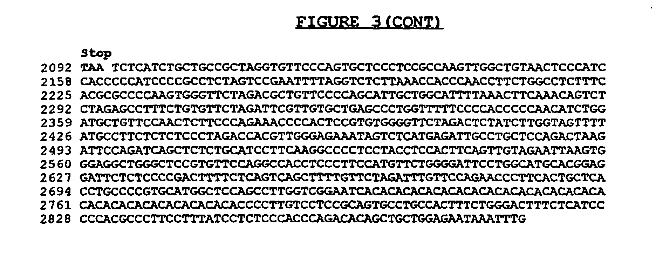

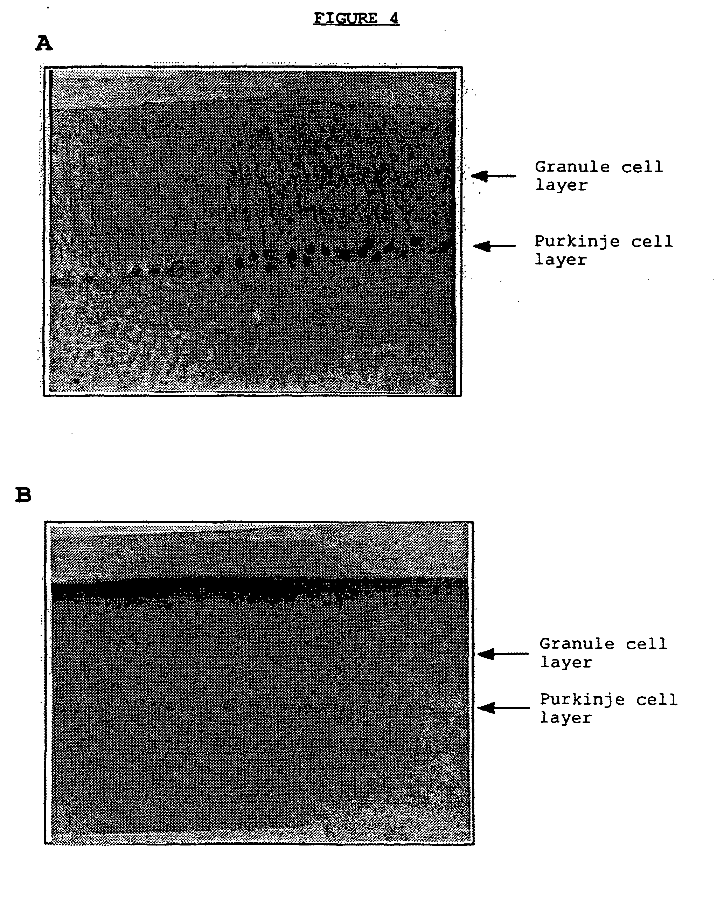

**Figure 5** illustrates a western blot of total brain proteins from the NNG3 and AS strains probed with an anti-PKCγ antibody. The lane marker (M) contains a BENCHMARK™ prestained protein ladder (GibcoBRL) and the sizes of the bands are indicated on the left-hand side. The lanes marked AS and NNG3 contain proteins isolated from brain tissue from AS and NNG3 rat strains, respectively; and

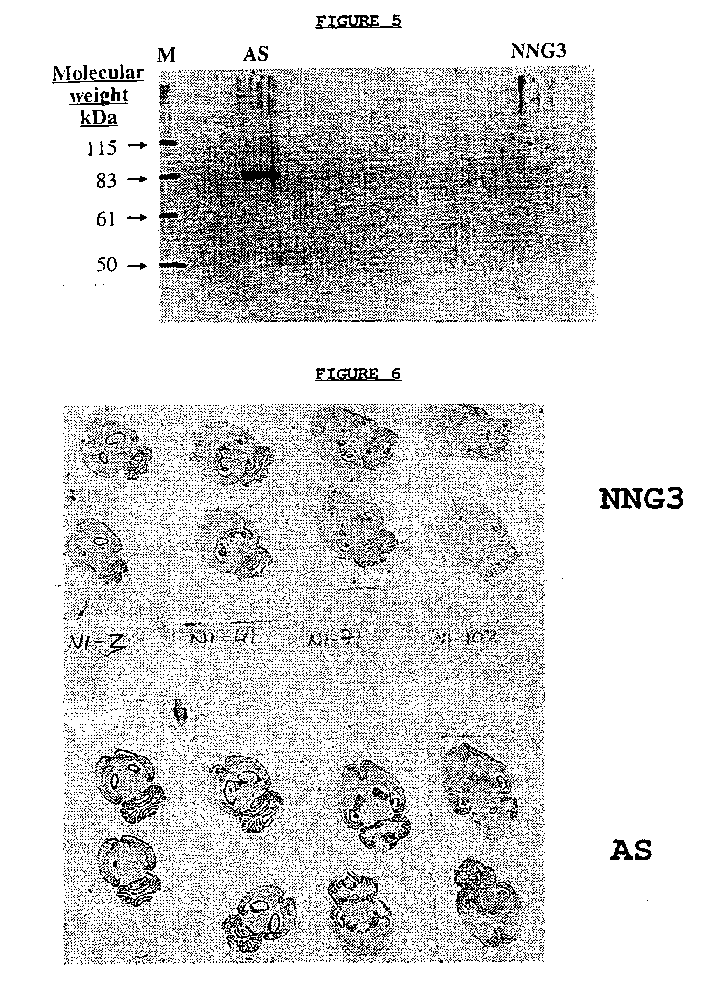

**Figure 6** illustrates an in situ hybridisation examination of the PKCγ mRNA transcript in the rat strains NNG3 and AS, as labelled. The brain sections from each strain were consecutive and all sections were probed simultaneously.

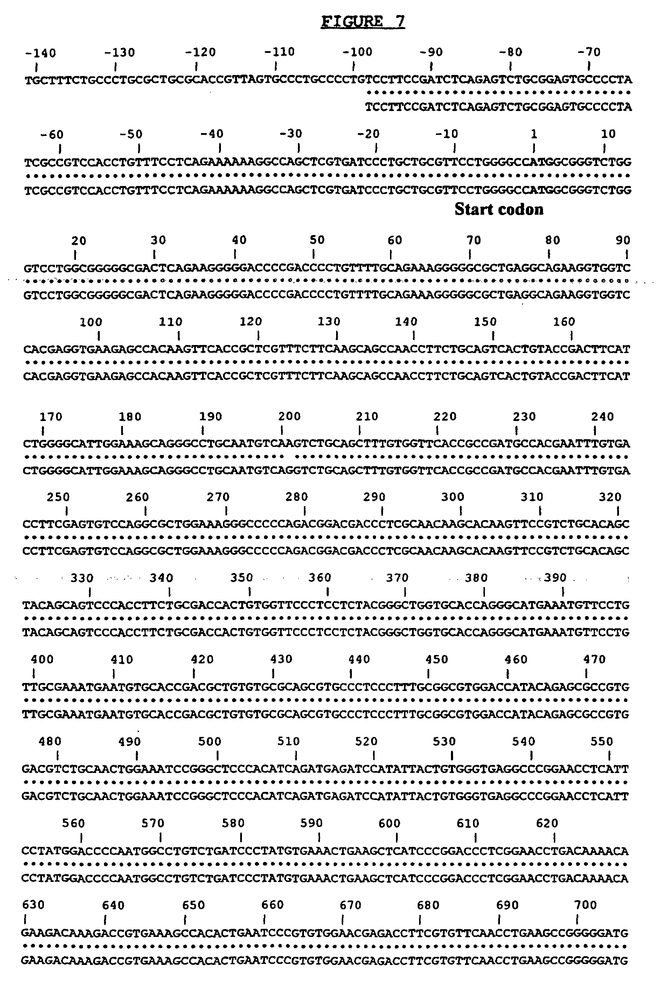

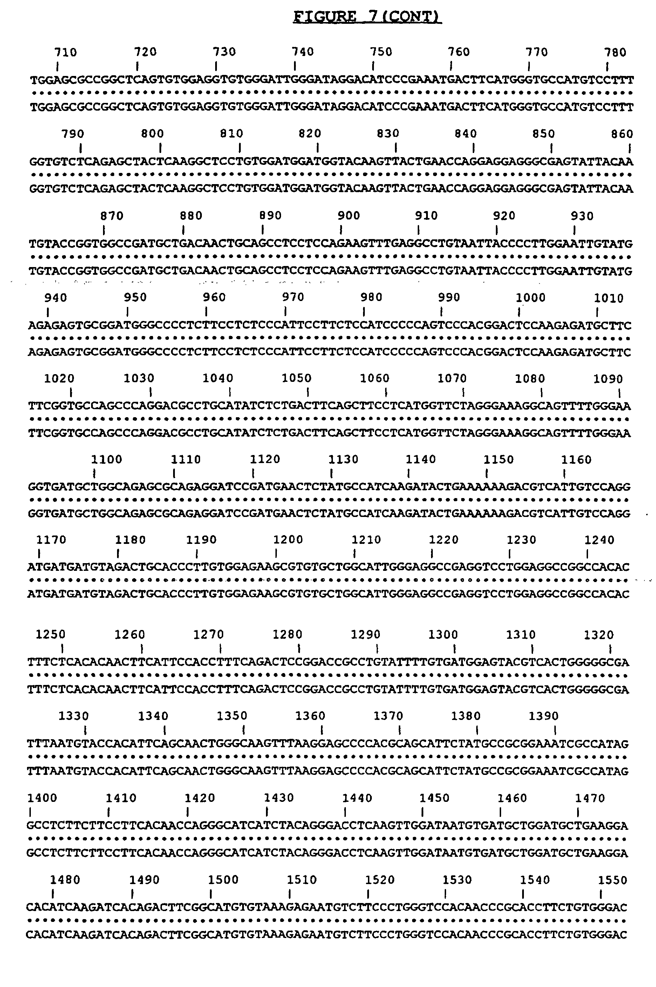

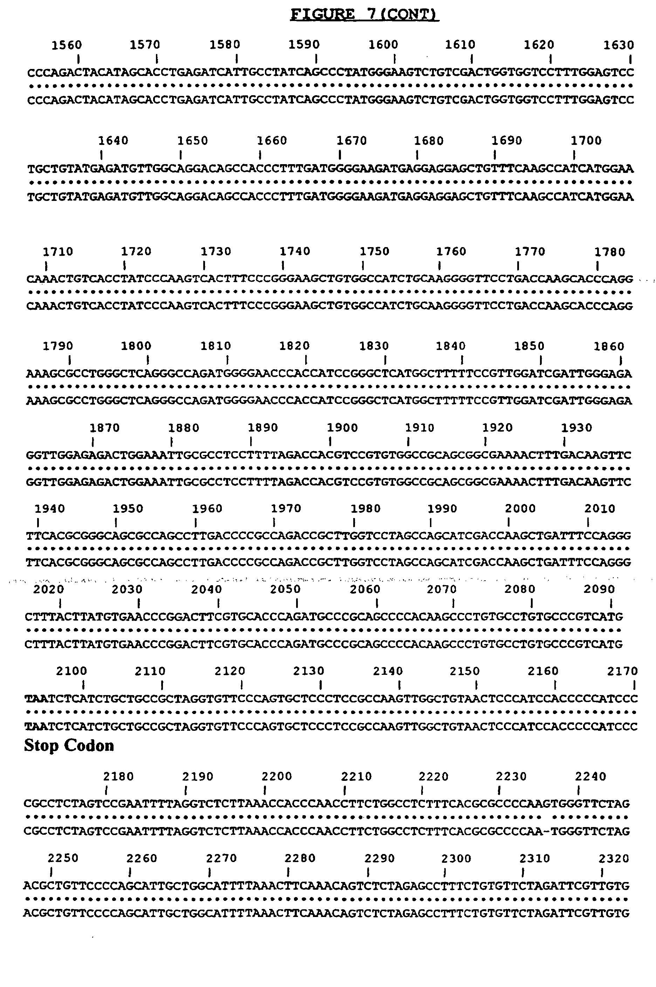

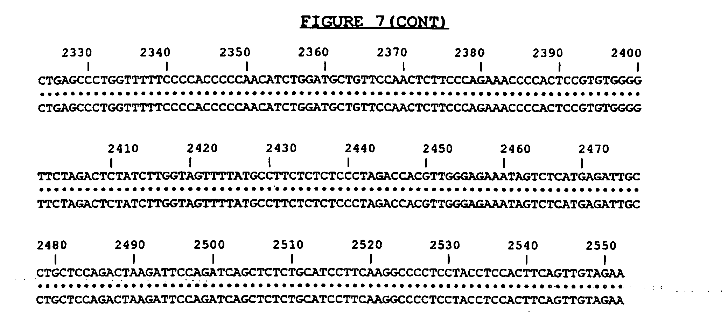

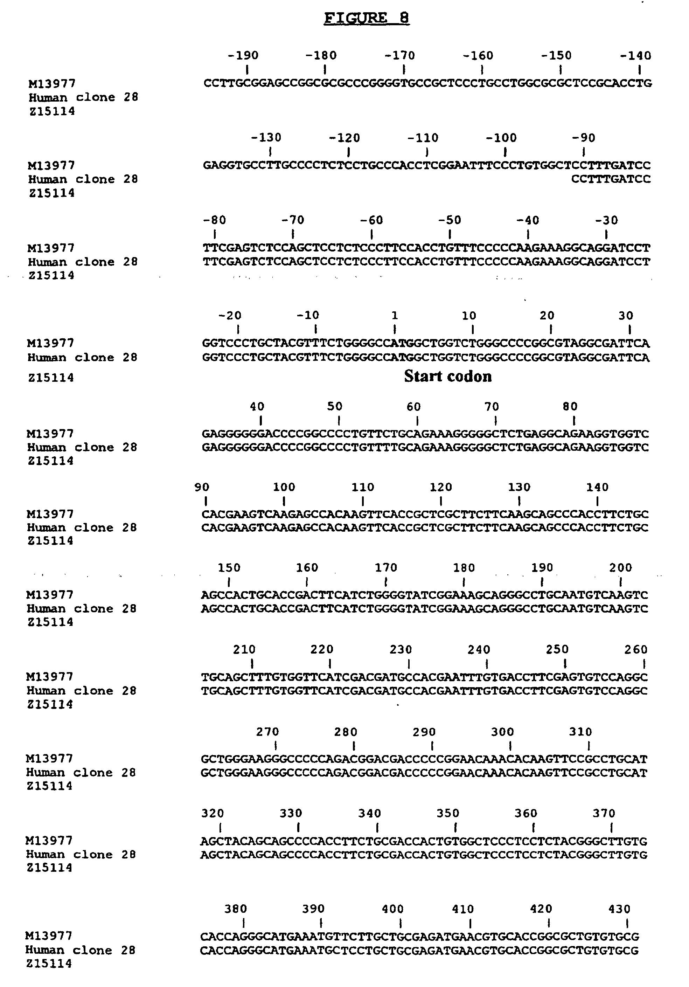

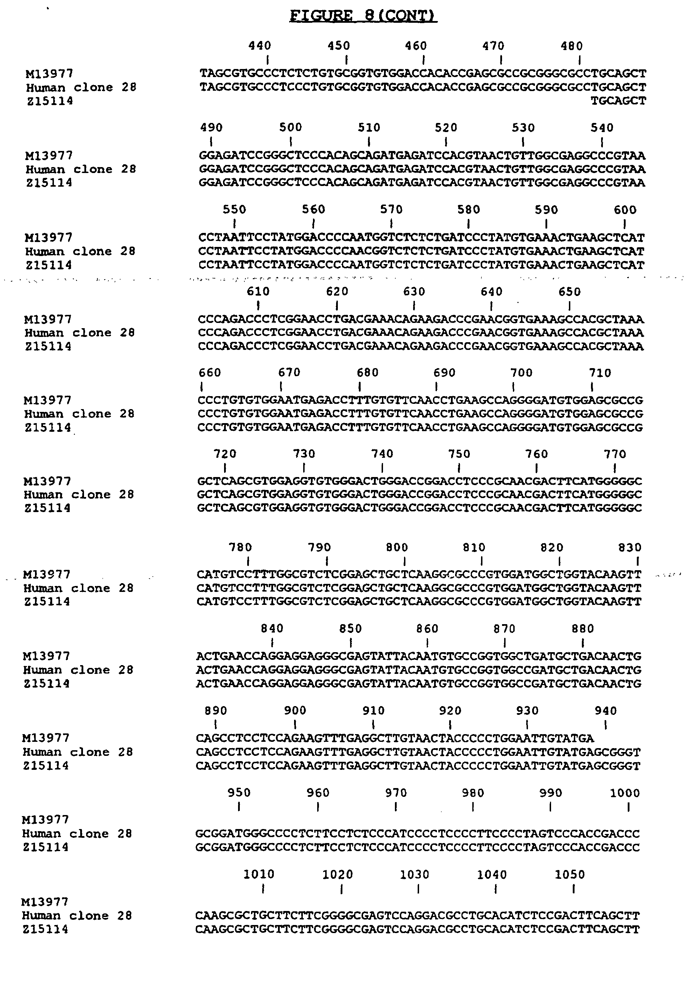

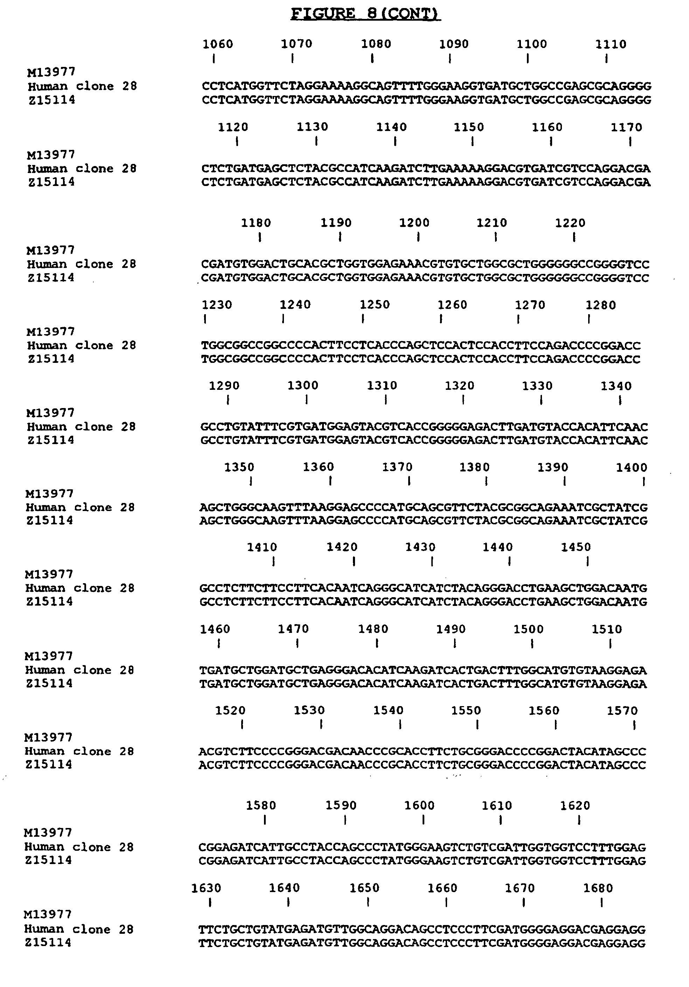

**Figure 7** illustrates an alignment of the AS Clone 17 sequence (bottom) and sequence from rat mRNA protein kinase C gamma from the NCBI database (Accession number: X07287) (top). A black dot between the sequences indicates identical bases. The start and stop codons are indicated in bold.

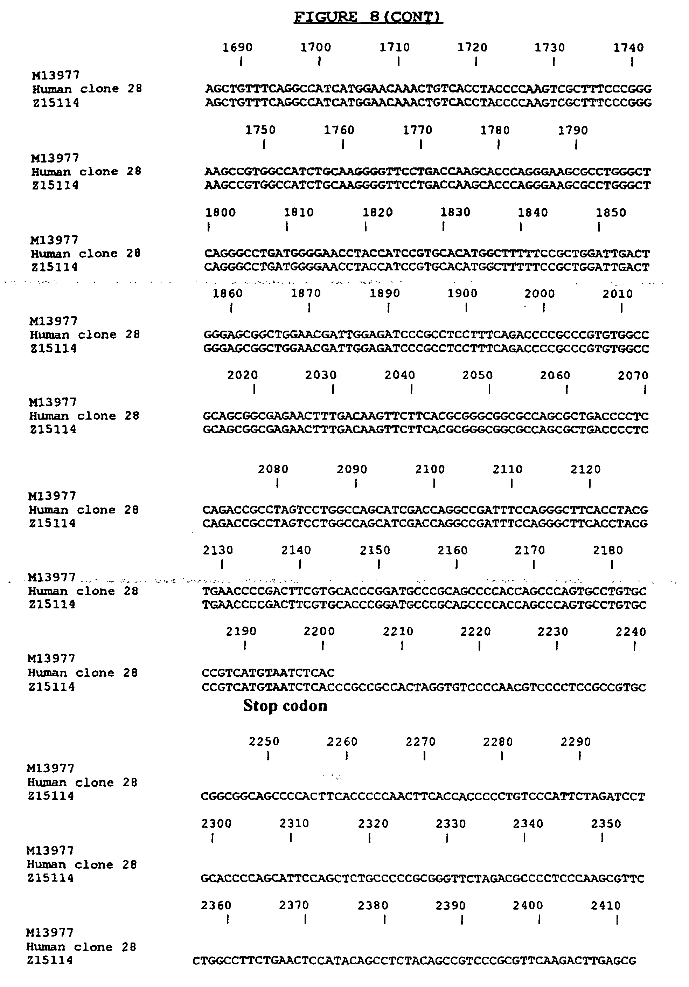

**Figure 8** illustrates an alignment of sequence obtained from the clone (Clone 28) of human full length cDNA, aligned to sequences obtained from the NCBI database (Accession numbers: Z15114, H.sapiens mRNA for protein kinase C gamma (partial) and M13977, Human (clone lambda-hPKC-gamma6) protein kinase C-gamma (PRKCG).

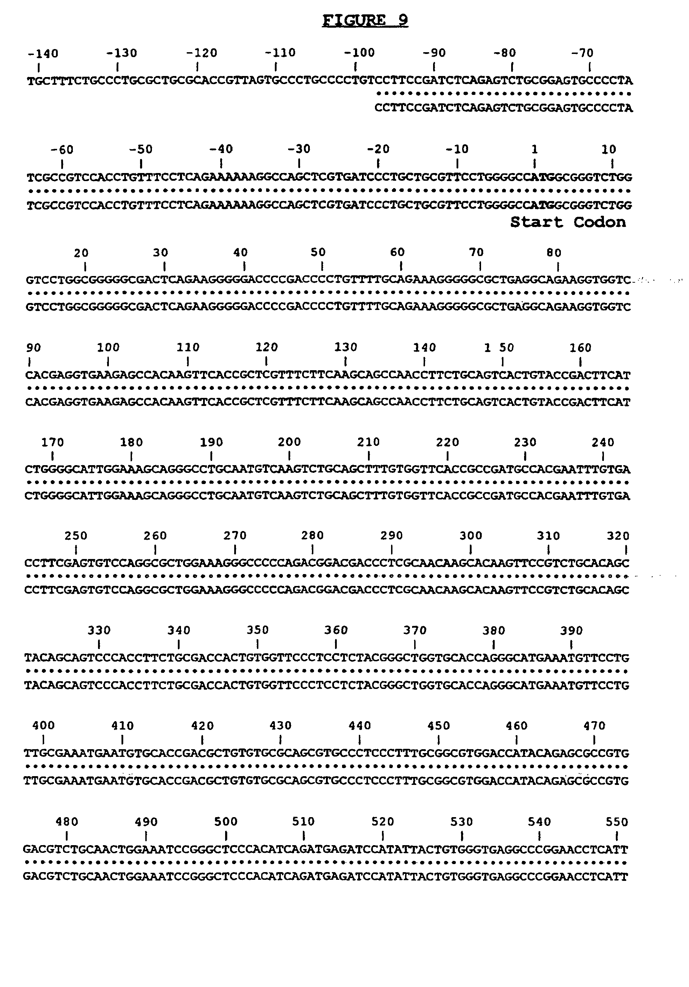

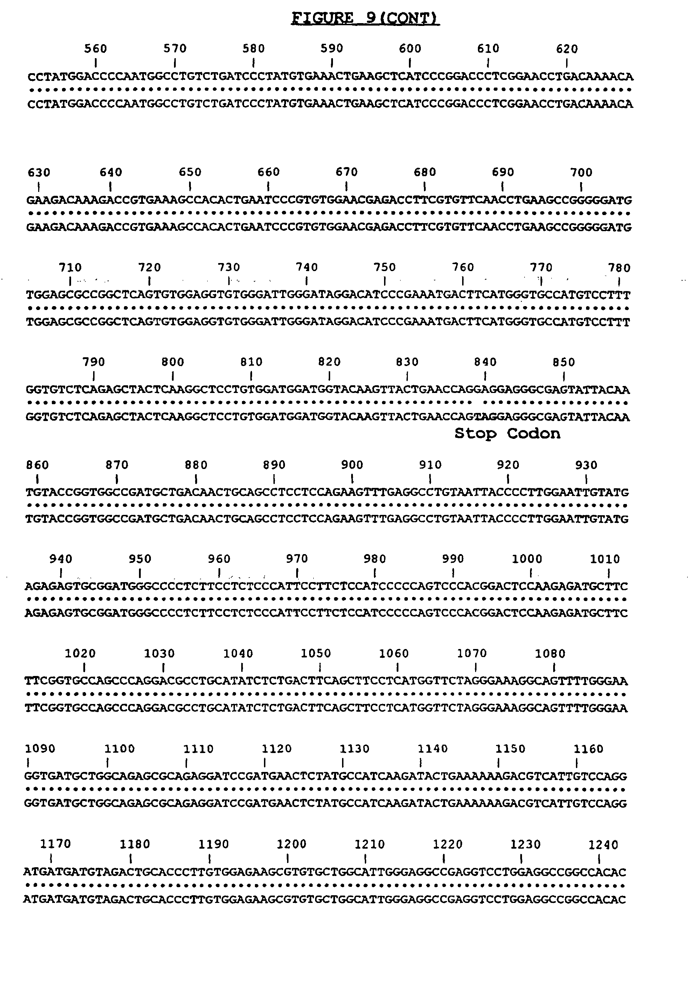

**Figure 9** is a comparison of the DNA sequence of rat PKCγ cDNA and NNG3 cDNA. The rat PKCγ sequence is on the top, the NNG3 sequence is on the bottom. The start and stop codons are shown in bold. There is a base error in the NNG3 sequence at base 1281, after the stop codon.

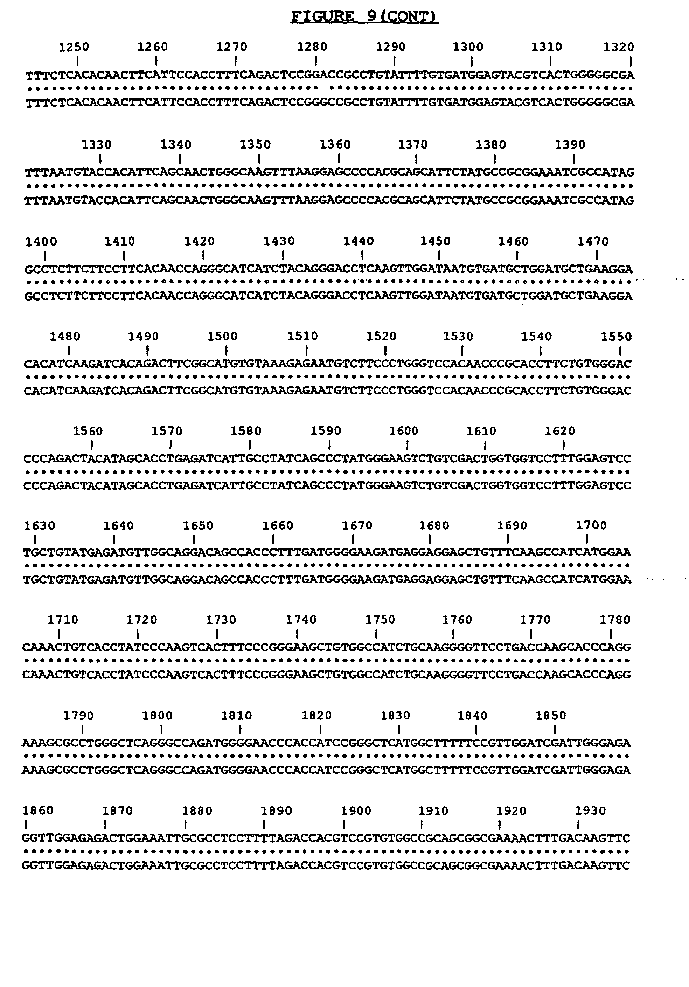

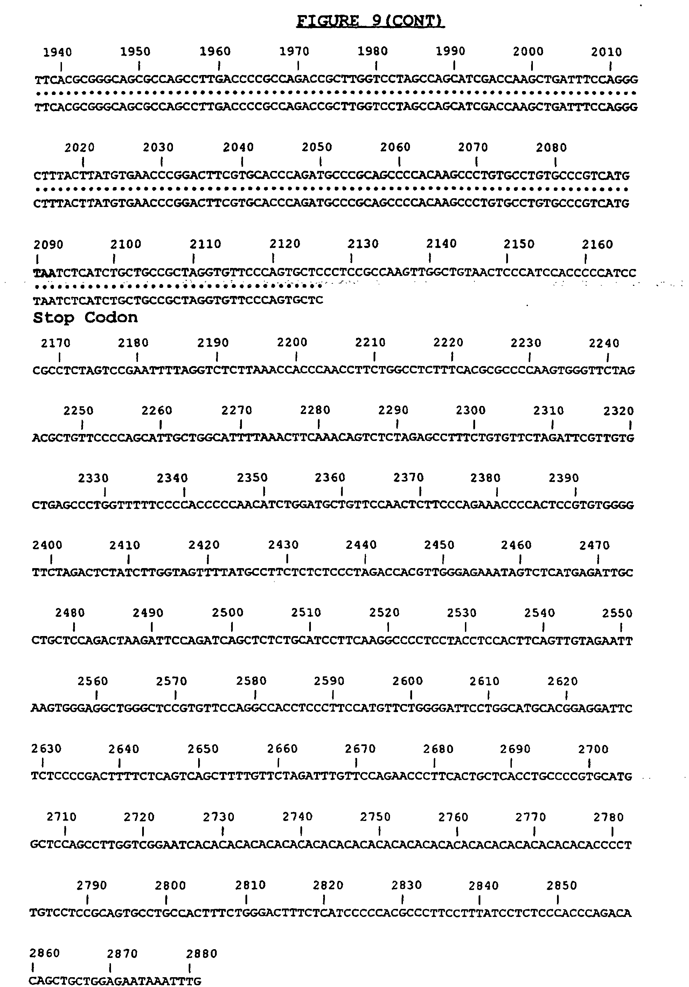

**Figure 10** is a comparison of the amino acid sequences of the translated PKCγ and NG3 cDNAs. The NNG3 sequence is shown on top whilst the rat PKCγ sequence is shown below.

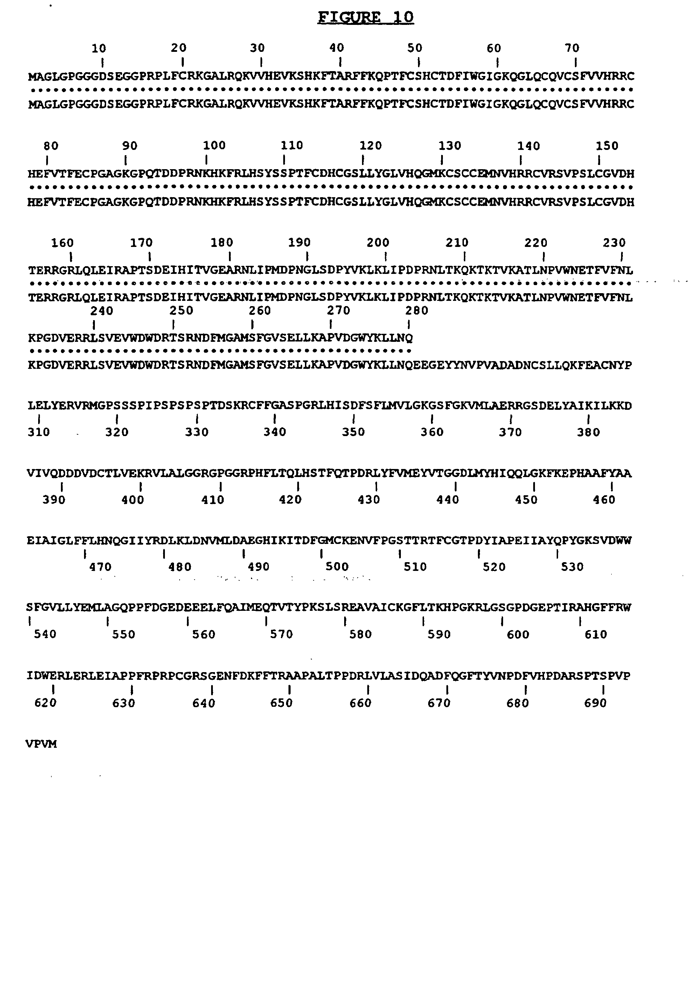

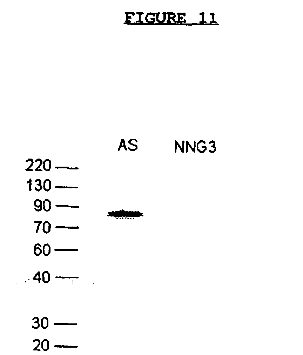

**Figure 11:** illustrates a Western blot of rat brain protein. (Primary antibody: RBI polyclonal PKC gamma at a dilution of 1:1000, Secondary antibody dilution: 1:1000).

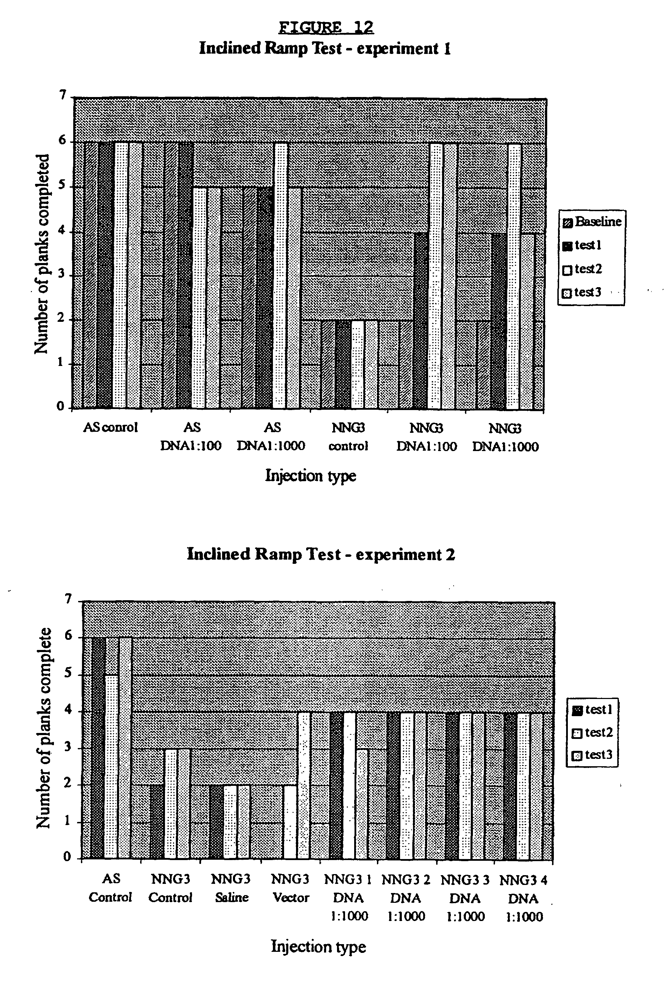

**Figure 12** illustrates the results of the inclined ramp test from two independent experiments. Plank 1 is 120mm wide, plank 2 is 100mm, plank 3 is 70mm wide, plank 4 is 50mm wide, plank 5 is 20mm wide and plank 6 is 10mm wide. The control animals were non-injected. The vector animal received pRcCMV2 without an insert. DNA refers to an injection of ratPKCγ in pRcCMV2. 1*µ*g of vector or DNA was delivered in PEI at a charge ratio of either 1:100 or 1:1000, as indicated.

### Experiment 1 - Genetic fine mapping of the nng3 mutation by genotyping of backcross progeny with R158

The present inventors applied genetic mapping techniques in order to ascertain the genotypic variation displayed in the AS/AGU strain using the process of "positional cloning" (Collins, 1992, Nature Genetics, 1, 3 - 6). Application of this approach relied upon initially determining the chromosomal localisation of the gene by demonstrating linkage to known marker genes. This was followed by additional fine mapping to narrow down the genetic region containing the gene, followed by either sequencing of the region or selection of mRNA transcripts from the region.

Genetic linkage is a direct consequence of the physical linkage of two or more genes with the same pair of DNA molecules that define a particular set of chromosome homologs within the diploid genome (Silver, 1995, Mouse Genetics: Concepts and Applications, Oxford University Press). Generally, crossing over occurs at random sites along all the chromosomes in the mammalian genome. A direct consequence of this randomness is that the further apart two linked loci are from each other, the more likely it is that a crossover event will occur some where within the length of chromosome which lies between them (Silver, 1995). Thus, the frequency of recombination provides a relative estimate of the genetic distance between a known marker gene and a previously unknown gene.

Three backcrosses were established with the NNG3 strain and strains F344, BN and DA (Festing, 1979 Inbred Strains in Biomedical Research, London : The MacMillan Press Ltd.) to allow mapping of the nng3 gene. The F344, BN and DA rat strains were chosen as they exhibited the highest variation within microsatellite sequences when compared with the NNG3 strain (Shiels et al, 1995, Mammalian Genome, 6, 214 - 215). Microsatellite sequences are defined as tandem repeats of simple dinucleotide or other DNA sequences which occur in allelic forms of various lengths. They are considered convenient genetic markers and are examined by assessing the length of a short polymerase chain reaction product containing the microsatellite using gel electrophoresis.

To establish a backcross, each strain in question (DA, F344 and BN) was crossed to the NNG3 strain and the resulting heterozygous F1 progeny were backcrossed to the NNG3 strain. The resulting backcross progeny were then genotyped to identify if a cross-over event had occurred between the gene of interest and any genetic marker. This allowed positioning of the gene to within a particular chromosome or sub-chromosomal region.

In total 3188 backcross progeny were produced from the three backcrosses. A whole genome scan was carried out with 73 microsatellite markers involving genotyping at least one informative marker per rat chromosome. All of these markers were assayed for linkage to the nng3 mutation. Genetic linkage was observed with the marker R33 (Serikawa et al, 1992, Genetics, 131, 701 - 721), which was localised to chromosome 1 and mapped approximately 30 cM from the nng3 gene. A range of markers from within this chromosomal region were then used to genotype all of the backcross progeny. The closest marker loci bracketing the nng3 mutation were chosen to establish a chromosomal interval within which to carry out precise mapping. This interval was different for each backcross, as shown in Figure 1. This allowed fine mapping of the region and identified recombination events between markers. The markers used are shown in Figure 1 for the three backcrosses (BN X NNG3) F1 X NNG3,(F344 X NNG3) F1 X NNG3 and (DA X NNG3) F1 X NNG3.

All progeny identified to have recombination events within the interval under investigation were genotyped using the genetic marker R158 (Serikawa et al, 1992). The genetic marker R158 consists of a pair of PCR primers which amplify a (CA)₂₆ microsatellite repeat from the 3'UTR of the PKCγ gene. The three rat strains BN, F344 and DA were shown to be informative for R158, that is, the length of the microsatellite was shown to vary between strains, when compared to the NNG3 rat strain. The genotyping was carried out by PCR, as described in the following experimental section, on genomic DNA. The PCR products were then resolved by gel electrophoresis either on 6% acrylamide or on 4% metaphor (Flowgen) agarose gels.

From this experiment (n=3188), no animals were observed to contain a recombination event between the nng3 mutation and the marker R158. This positioned the nng3 mutation 0 ± < 0.06 cM from the genetic marker R158. In the mouse, 0.06 cM corresponds to approximately 60 kb of chromosome DNA length. These mapping experiments show that the nng3 mutation (and gene) is very close to the R158 microsatellite, which is itself within the gene PKCγ encoding the type I isoform of protein kinase C. Thus, the nng3 gene is very likely to be PKCγ itself or an immediately adjacent gene.

### Experiment 2 - Demonstration of a DNA sequence difference between the allelic forms of the PKCγ gene in the strain and the parent AS strain

The genetic mapping evidence implicated the PKCγ gene as the location of the nng3 mutation. If this implication was correct, a DNA sequence difference must have existed between the allelic forms of the PKCγ gene in the NNG3 strain and in the AS strain from which the NNG3 strain arose by spontaneous mutation. Thus, the following experiment was performed to provide evidence of the sequence difference.

RNA was isolated from 12 month old rats from both the mutant (NNG3) and control (AS) strains. RNA was isolated using TRI REAGENT™ (Sigma) as per the protocol supplied by the manufacturer. RNA was isolated from 1g of brain tissue and homogenised in 10 ml of TRI REAGENT™. 1*µ*g of RNA was then used to synthesise cDNA using the Oligo (dT)₁₂₋₁₈ Superscript™ Preamplification System for First Strand cDNA Synthesis kit from Gibco BRL. 50ng of cDNA was then used as template in a PCR reaction. The PCR reaction was carried out using 1 unit of Taq DNA polymerase (Promega) in a reaction containing 1x magnesium free Thermo buffer, 1mM magnesium chloride (all supplied with Tag polymerase from Promega), 125*µ*M dNTPs (Promega) and forward and reverse primers at 5ng/*µ*l each. A hot start was always performed in the PCR reaction. The sequences of all PCR primers used are given in Table 1, along with the PCR reactions in which they were used.

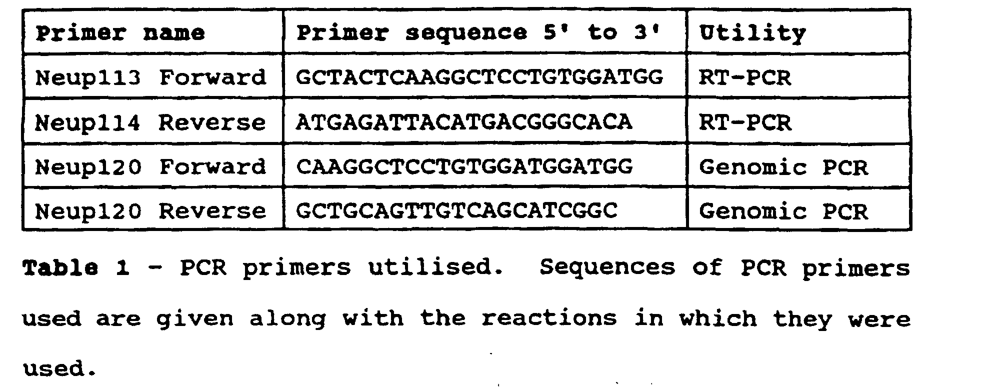

The PCR parameters used for Taq DNA polymerase were as follows:

| 113 Forward/114 Reverse | 120 Forward + Reverse |
|---|---|
| 99°C - 3 mins | 99°C - 10 mins |
| 80°C - Taq added | 80°C - Taq added |
| Followed by 35 cycles of: | Followed by 30 cycles of: |
| 94°C - 15 secs | 94°C - 15 secs |
| 55°C - 30 secs | 55°C 30 secs |
| 72°C - 1 min. Followed by: | 72°C - 30 sees. Followed by: |
| 72°C for 10 mins. | 72°C - 10 mins. |

PCR products were then resolved on a 2% agarose gel (Boehringer Mannheim) and then extracted from the gel using the Qiaquick™ gel extraction kit prior to sequencing.

Sequencing of the PCR products was carried out on the ABI 373 stretch automatic sequencer using the Big Dye terminator chemistry (Perkin Elmer). 3.2 pmoles of a primer used in the PCR reaction were used for sequencing.

PCR reactions were also carried out on rat genomic DNA isolated from rat spleen taken from both rat strains. DNA was isolated using the Pure Gene DNA Isolation kit (Gentra). PCR reactions were carried out as before containing 100 ng of genomic DNA as template. The initial denaturation step of the PCR reaction was also increased to 10 minutes.

PCR reactions were also carried out using proof-reading DNA polymerase enzymes to eradicate errors during DNA polymerisation. The enzymes and modifications of the PCR reactions were as follows: *Tli* polymerase (Promega) reactions were carried out with 1.25 units of the enzyme, which extends at 74°C. The High Fidelity (HF) kit from Clontech has an automatic hot start and anneals and extends concurrently at 68°C for 3 minutes.

Three different populations of cDNA were synthesised and PCR products obtained from these sequences (a summary of experimental results is given in Table 2). All PCR products sequenced exhibited a G nucleotide in the AS rat strain at position 841 and a T nucleotide at the same position in the NNG3 rat strain, as shown in Figure 2. This transversion mutation creates a new termination or stop codon for translation of PKCγ, resulting in a prematurely terminated protein (Figure 3). As the kinase active domain of the protein is lost, this will result in a loss of ability to phosphorylate the target protein substrates, leading to a cascade of biological consequences. This proposed loss of function appears to be in agreement with the recessive nature of the mutation.

**Table 2 -**

| Summary of sequencing experiments carried out. The table details the enzymes and templates used in PCR reactions carried out for sequencing. The nucleotide number referred to in the table (841) is taken from Figure 2. | | | |
|---|---|---|---|
| **Experiment no.** | **Template** | **PCR Enzyme** | **Summary** |
| 1 | cDNA: 1 | Taq | G at position 841 in AS and T at 841 in NNG3 |
| 2 A: B: | cDNA: 2 cDNA: 1 | Taq Taq | G at position 841 in AS and T at 841 in NNG3 |
| 3 | cDNA: 2 | Tli (proof-reading) | G at position 841 in AS and T at 841 in NNG3 |
| 4 | cDNA: 2 | HF-kit (proof-reading) | G at position 841 in AS and T at 841 in NNG3 |
| 5 | cDNA: 3 | HF-kit | G at position 841 in AS and T at 841 in NNG3 |
| 6 | Genomic DNA | Taq | G at position 841 in AS and T at 841 in NNG3 |
| 7 | Genomic DNA | Tli | G at position 841 in AS and T at 841 in NNG3 |

These mapping and sequencing results demonstrate that a sequence difference does appear to occur between the NNG3 and AS strains. It is conceivable that this base change (G to T at position 841, Figure 2) in the NNG3 strain gives rise to the phenotype observed in this strain.

### Experiment 3 - Detection of PKC gamma by immunocytochemistry

It was postulated that the stop codon in the PKCγ gene in the NNG3 strain led to premature termination of the PKC type I polypeptide. This would result in the protein having no kinase activity. The binding of antibodies raised against the carboxy terminal portion of the PKC type I protein (Boehringer Mannheim) to brains isolated from the NNG3 strain were thus investigated.

PKC gamma was detected by immunocytochemistry using standard protocols with the following modifications: rat brains were taken from 9 months old male rats and were fixed overnight in 4% paraformaldehyde. Brains were then trimmed, placed on a block and sections cut at 50 microns using a vibratome. Paired sections were placed into blocking serum (10% NGS - normal goat serum) and placed on shaker for 1 hour at room temperature. Sections were then placed into primary antibody (rabbit anti-peptide antibody generated using a synthetic peptide corresponding to amino acids 306 - 318 of rat PKCγ, 1:100 - 1:2000, Boehringer Mannheim) overnight at 4°C. Slides were then washed three times in PBS for 5 mins each. Sections were then placed into secondary antibody (biotinylated sheep anti-rabbit IgG antibody, Vector) for 1 hour. Sections were again washed three times in PBS (5 mins each). Vecta stain ABC complex (Vector) was then added to the sections for 1 hour and placed on a shaker. Sections were again washed three times in PBS (5 mins each) and then washed once in PB for 5 mins. 3,3'Diaminobenzidine tetrahydrochloride (DAB) was applied to the sections for 5-10 mins. Sections were washed twice in PB (5 mins each) and the sections finally dehydrated, cleared and mounted.

Figures 4(a) and (b) illustrate the difference between the NNG3 strain and the AS parent strain, from which NNG3 is derived.

A very striking depletion of PKCγ positive cells in Purkinje cells of the NNG3 strain was observed when compared to an age matched AS control. The Purkinje cell layer is a region where PKCγ is predominantly expressed when compared to other PKC isoforms.

These results are consistent with a loss of the carboxy-terminal part or all of the PKC type I protein.

### Experiment 4 - Western blot detection of Type I PKC in rat brain protein extracts by antibody hybridisation

A Western blot experiment was carried out to determine the level of expression of the Type I PKC protein in brains from the rat strains AS and NNG3. Specifically, antibodies raised against a peptide located in the carboxy terminal to the truncation site in the PKCγ type I protein of the NNG3 strain were used in order to confirm the lack of expression of this region of the protein.

Total brain proteins were extracted from male, 9 month old rats from both the AS and NNG3 stains. The proteins were isolated from 0.2g of brain tissue using TRI-REAGENT™ (Sigma) and suspended in 2% SDS. 50*µ*g of total proteins were resolved on a 10% SDS-PAGE (polyacrylamide gel electrophoresis) gel at 200V for 45 minutes. The proteins were then transferred to nitrocellulose (Amersham Life Science) in a wet Western blotter at 30V overnight. The nitrocellulose was then blocked for 1 hour at room temperature, in a solution of Tris-buffered saline (20 mM Tris-HCl, pH 7.5, 150 mM NaCl) containing 0.05% Tween 20 (TEST) and 1% bovine serum albumin (BSA). The nitrocellulose was then incubated in TBST plus 1% BSA containing 2 *µ*g/ml anti-PKCγ (Rabbit anti-peptide antibody generated using a synthetic peptide corresponding to amino acids 306-318 of PKCγ, GibcoBRL). The blot was then washed 3 times in TBST for 10 minutes each. The blot was incubated for one hour at room temperature in TBST containing anti-rabbit Ig, peroxidase-linked species-specific whole antibody (from donkey, Amersham Life Science) at a dilution of 1/1000. The blot was then washed as detailed above. After washing the blot was incubated with ECL Western blotting detection reagents (Amersham Pharmacia biotech) and then exposed to autoradiography film (Fuji XR).

This experiment was performed on proteins extracted from the NNG3 strain and the control rat strain (AS). The results obtained are illustrated in Figure 5. A band of the predicted size (80 kDa) was obtained from the AS strain protein extract, whereas no signal was obtained in the NNG3 strain protein extract. The anti-PKCγ antibody recognises an epitope corresponding to amino acids 306-318 of the protein. In NNG3 it is postulated that a truncated protein is produced which terminates at amino acid 281; therefore, these results illustrate that the epitope for antibody binding is not present in the NNG3 protein, as predicted.

### Experiment 5 - In situ hybridisation investigation of the PKCγ mRNA transcript in AS and NNG3 rats

An *in situ* hybridisation experiment was carried out to determine the level of expression of the mRNA encoding Type I PKC in the rat strains AS and NNG3.

An antisense oligonucleotide probe was designed to the 3' region of the PKCγ MRNA (nucleotides 2085-2326, numbers taken from Figure 2) and was synthesised and purified by HPLC (GibcoBRL). The sequence of the oligonucleotide was as follows:

5' GCA CTG GGA ACA CCT AGC GGC AGC AGA TGA GAT TAC ATG ACG
3'

Whole brains were taken from 8 month old AS and NNG3 rats. These brains were mounted and sectioned horizontally in 13 micron sections and the sections thaw-mounted onto poly-L-lysine treated microscope slides. The sections were then fixed in 4% paraformaldehyde in 1x PBS (phosphate buffered saline, all solutions were made with (diethylpyrocarbonate-treated water) on ice for 5 minutes, followed by PBS for 2 minutes, then dehydrated in 70% ethanol for 2 minutes, 95% ethanol for 5 minutes and stored in 100% ethanol at 4°C until required.

The in situ hybridisation probes are labelled and prepared for hybridisation in the following way: 40ng of the oligonucleotide was labelled in a reaction volume of 12.5*µ*l made up in DEPC-water containing 1x reaction buffer (supplied with enzyme), 25*µ*Ci of S³⁵ α-dATP and 36 units of Terminal deoxynucleotidyl transferase (TdT, FPLC pure, Pharmacia). The reaction was then incubated at 37°C for 1 hour. Purification columns were constructed in 1 ml plastic syringes with GF/C filter paper, cut with a No. 2 cork borer, placed at the bottom of the syringe and the syringe packed with G50 Sephadex (Pharmacia). The column was then centrifuged at 2,000 rpm for 1 min to pack the Sephadex. 87.5 *µ*l of DEPC-treated water was then added to the probe to make to 100 *µ*l. The probe was added to the column and centrifuged for a further 1 minute at 2,000 rpm in a 1.5 ml centrifuge tube for collection. The eluant was then collected and the volume measured. The specific activity of the probe was determined by placing 2 *µ*l of the eluted probe in a scintillation vial and adding 5 ml of scintillant. The specific activity of the probe was then measured in a scintillation counter uwing the Tritium channel. The probe was then standardised so as produce 2 x 10³ disintegration's per minute/ml of hybridisation mix 50% formamide, 4 x SSC, 10% dextran sulphate, 5 x Denardt's, 200µg/ml acid-alkali cleared salmon sperm DNA, 100µg/ml long chain polyadenylic acid, 120µg/ml heparin, 25mm sodium phosphate, pH7, 1mm pyrophosphate, then DDT added to a final concentration of 20mm. A 100 times excess of cold oligonucleotide was also added to the control hybridisation mix.

Serial sections were selected for hybridisation so as to be pair-matched and spread throughout the brain. The sections were covered with 200 *µ*l of the appropriate hybridisation mix and then covered with a parafilm coverslip. The slides were then incubated overnight at 42°C. The following day the coverslips were floated off in 1 x SSC at room temperature and the sections were washed twice in a shaking water-bath at 55°C in 1x SSC containing 4 mM DTT for 30 minutes. The sections were then dehydrated through 1x SSC for 30 seconds, 0.1x SSC for 45 seconds, then 70% ethanol for 2 minutes and finally 100% ethanol for 5 minutes. The slides were then allowed to air dry. Once dry the slides were taped to 3MM filter paper and exposed to autoradiography film (Kodak Bio-max MR film, single coated) at room temperature for 1 week.

The results of the *in situ* hybridisation experiment are illustrated in Figure 6. It is evident that the level of PKCγ mRNA in the NNG3 strain appears to be altered when compared to age-matched control brains from the AS strain.

### Experiment 6 - Isolation of PKCγ cDNAs from rat and human

Full length cDNA clones were isolated for the PKCγ gene, by reverse transcriptase-polymerase chain reaction (RT-PCR) amplification, from RNA isolated from both the rat strain Albino Swiss (AS) and human. The brain was removed from a 12 month old male AS rat after decapitation and 1g of tissue removed. Total RNA was isolated from the tissue using TRI REAGENT™ (Sigma, Poole, Dorset) as per the protocol supplied by the manufacturer. Human brain RNA from a normal individual was obtained from Stratagene. 1 *µ*g of RNA from each was then used to synthesise cDNA using the Oligo (dt)₁₂₋₁₈ Superscript™ Preamplification System for First Strand cDNA synthesis kit from Gibco BRL (Inchinnan Business Park, Paisley). 50 ng of cDNA was then used as template in PCR reactions. All PCR reactions were carried out using the Clontech Advantage®-HF PCR kit as per the manufacturers instructions (Basingstoke, Hampshire). The full length AS cDNA was amplified using the primers Neup 118F 5' -CCT TCC GAT CTC AGA GTC TGC GG- 3' and K3R 5'-TTC TAC AAC TGA AGT GGA GG-3' (PCR parameters: 94°C for 15 seconds, 25 cycles of 94°C for 10 seconds, 64°C for 4 minutes, followed by 68°C for 3 minutes). The full length human cDNA was amplified using the primers Neup 144F 5'-TGA TCC TTC GAG TCT CCA GC-3' and Neup 144R 5'- ACG TTG GGG ACA CCT AGT GG-3' (PCR parameters: 94°C for 15 seconds, 25 cycles of 94°C for 10 secs, 68°C for 3 minutes, followed by 68°C for 3 minutes). The amplified fragments were cloned using the Zero Blunt® ToPO® PCR cloning kit (Invitrogen, CH Groningen, Netherlands).

Sequence analysis was then carried out on the clones to investigate any errors introduced during PCR amplification. Sequencing reactions were carried out using the BigDye terminator kit (PE Biosystems, Birchwood science park, Warrington) in the presence of 3.2 pmoles of a primer used in the PCR reaction. The reactions were carried out in a Perkin Elmer GeneAmp PCR system 9600 (Perkin Elmer, Birchwood science park, Warrington) as follows: 96°C for 2 minutes, 25 cycles of 94°C for 10 seconds, 50°C for 5 seconds and 60°C for 4 minutes. The sequencing reactions were resolved on an ABI 373 stretch automatic DNA sequencer. Sequence data was analysed using Seqed software (PE Biosystems).

Sequencing results demonstrated that there was one base change identified in the AS sequence when compared to the sequence lodged in the database (NCBI Accession number: X07287). This base change CAA->CAG (base 201, Figure 7) did not change the amino acid sequence and may simply indicate a polymorphism. There is also a G which is not present in AS when compared to the mRNA protein kinase C gamma (base 2236). This base deletion occurs after the stop codon and may also represent a species difference.

Some differences were also observed in the human cDNA clone when compared to the two sequences lodged in the NCBI database (Table 3 and Figure 8), but again these did not change the amino acid and may indicate polymorphism. Indeed one of these polymorphisms had previously been described by Al-maghtheh, *et.al.,* (Al-Maghtheh, M., Vithana, E.N., Inglehearn, C.F., Moore, T., Bird, A.C. and Bhattacharya, S.A. (1998). Segregation of a PRKCG mutation in two RP11 families. American Journal of Human Genetics, 62, 1248-1251), AAT189AAC. The two sequences lodged in the database were also polymorphic at this base.

**Table 3 -**

| Base changes identified within Human clone 28 when compared to the sequence lodged in the NCBI database (Accession numbers: Z15114, H.sapiens mRNA for protein kinase C gamma (partial) and M13977, Human (clone lambda-hPKC-gamma6) protein kinase C-gamma (PRKCG). | | |
|---|---|---|
| **Base number (Fig 8)** | **Base Change** | **Amino acid Conserved** |
| 56 | TTC->TTT | Phe |
| 392 | TGT->TGC | Cys |
| 395 | TCT->TCC | Ser |
| 444 | TCT->TCC | Ser |
| 567 | AAT->AAC | Asn (Al-maghtheh, 1998 AAT189AAC) |
| 873 | GCT->GCC | Ala |

### Experiment 7: Cloning of the NNG3 PKCγ cDNA

PCR of the full length cDNA had been attempted, but failed. Therefore, PCR amplification of the 5' and 3' ends of the NNG3 cDNA, in two separate reactions was performed.

### 5' rapid amplification of cDNA ends (RACE)

Total RNA from NNG3 rat brain was prepared using Tri-reagent (Sigma) according to manufacturers instructions. 1mg of this total RNA was used with the 5' RACE system (Gibco BRL). The protocol supplied with the kit was followed. Gene specific primer 104R (5' - CTTA AACTTGCCCAGTTGCT - 3') was used as the GSP1. This corresponds to the antisense sequence between bases 1480 and 1500 of the PKCγ cDNA (see figs 7 and 9). The PCR reaction was conducted using 0.4 *µ*l of the high fidelity enzyme, Expand (Roche Diagnostics Ltd, Lewes, East Sussex), with 1*µ*l 10mM dNTPs, 5*µ*l template cDNA, 5*µ*l 10 x expand buffer, 3*µ*l 25 mM MgCl₂, 1*µ*l of 10*µ*M abridged anchor primer (5' -GGCCACGCGTCGACTAGTACGGGIIGGGIIGGGIIG - 3') and 1*µ*l of 1*µ*M GSP1. The following conditions were employed for the PCR, using a Perkin Elmer GeneAmp PCR system 9600: 94°C for 2 minutes then 30 cycles of 94°C for 15 seconds, 50°C for 30 seconds and 72°C for 2 minutes and finally 7 minutes at 72°C.

The product of this PCR was a smear. Therefore the DNA was diluted 1:500 in T/E (10mM Tris pH 8.0, 0.1 mM EDTA) and a second round of PCR was performed. The same reaction conditions as above were used, but the abridged anchor primer was replaced with the gene specific primer 118F (5' - CCTTCCGATCTCAGACT - 3').

A product of the expected size (1.3kB) was obtained from this round of PCR and it was directly cloned into the vector pCRbluntIITOPO using the Zero Blunt® TOPO® PCR cloning kit (Invitrogen) and manufacturers directions. It was named NNG3 5/12.

### 3' RACE

Total RNA from NNG3 rat brain was prepared using Tri-reagent (Sigma) according to manufacturers instructions. 1mg of this total RNA was used with the 3' RACE system (Gibco BRL). The protocol supplied with the kit was followed.

The PCR reaction was performed using the above conditions and components but replacing the primers with the universal amplification primer (5'-CUACUACUACUAGGCCACGCGTCGACTAGTAC - 3') and the gene specific primer 113F (5' - GCTACTCAAGGCTCCTGTGGATGG - 3') corresponding to the region 793 to 816 bases of the PKCγ cDNA (see figs 7 and 9).

Again the product of this PCR was a smear. Therefore the DNA was diluted 1:500 in T/E and a second round of PCR was performed. The same reaction conditions as above were used, but the universal amplification primer was replaced with a second gene specific primer 114R (5' - TGAGATTACATGACGGGCACA - 3') corresponding to the region antisense between bases 2077 and 2096 of the PKCγ cDNA (see figs 7 and 9).

A product of the expected size (1.3kB) was obtained from this round of PCR and it was directly cloned into the vector pCRbluntIITOPO using the Zero Blunt® TOPO**®** PCR cloning kit (Invitrogen) and following manufacturers directions. It was named NNG3 3/9.

The two NNG3 clones provide overlapping sequence between bases 797 and 1357. A single BamHI restriction digest site at 1112 bases was therefore used to join the two fragments together.

NNG3 5/12 and NNG3 3/9 were digested with HindIII (Promega) and BamHI (Promega) to release the 5' NNG3 fragment from both plasmids. The NNG3 5' fragments was purified from the NNG3 5/12 digest whilst the plasmid was purified from the NNG3 3/9 digest. Agarose gel purification was performed using a Qiaquick Gel Extraction kit (Qiagen).

The fragment purified from NNG3 5/12 could then be ligated into NNG3 3/9 to give the full length NNG3 cDNA. The DNA was then sequenced to check assembly and to ensure that no mutations had been introduced by PCR.

The two proteins were then translated using Seqed software (PE biosystems) and it could be seen that the NNG3 protein is terminated at amino acid 280 (see Figure 10).

### Experiment 8 - Western blot of PKCγ

Brain protein extracts from normal AS rats and NNG3 rats were prepared by homogenising 500mg of tissue in 2.5ml of ice cold 0.2 M potassium phosphate buffer, pH 7.6. The homogenates were centrifuged at 1000g for 15 minutes at 4°C. The supernatant from each sample was carefully removed with a syringe and stored at -20°C in aliquots.

The protein concentration of each sample was measured using a bicinchinic acid protein assay kit (Sigma). 30*µ*g of each protein sample, combined with an equal volume of sample buffer (0.0625M Tris, pH 6.8, 2% sodium dodecyl sulphate, 10% glycerol, 5% β-mercaptoethanol, 0.001% bromophenol blue), was then loaded onto a 10% polyacrylamide gel (28.2:0.8 acrylamide:bisacrylamide, 0.375 M Tris, pH 8.8, 0.1% sodium dodecyl sulphate) with a 3% stacking gel (28.2:0.8 acrylamide:bisacrylamide, 0.125 M Tris, pH 6.8, 0.1% sodium dodecyl sulphate). Prestained Benchmark protein standards (Gibco BRL) were loaded alongside the protein samples to provide a reference for the molecular weight of the protein of interest. Running buffer (0.025 M Tris (base), 0.192 M glycine, 0.1% sodium dodecyl sulphate) was added to the mini-gel apparatus (Biorad, Hemel Hempstead, Hertfordshire) and a voltage of 200V was placed across the gel, using a Biorad Power Pack 300 electrophoresis power supply unit.

After 45 minutes the dye front had reached the bottom of the gel, indicating that the electrophoresis of the gel was complete. The gel apparatus was dismantled and the gel was placed in a western blotting apparatus (Biorad) against a piece of PVDF membrane (Roche). The proteins were transferred onto the membrane using Towbin buffer (12.11.g Tris (base), 57.6 g Glycine, 800ml methanol, 1.2 ml hydrochloric acid in a volume of 4 l) and a voltage of 40v, applied over night.

The membrane, with bound proteins, was removed from the apparatus and submerged in blocking solution (10% (w/v) dried milk in PBS-T (8 g NaCl, 0.2 g KCl, 1.44 g Na₂HPO₄, 0.24 g KH₂PO₄ adjusted to pH 7.4 and made up to 11, with 0.1% tween 80) for 30 minutes. A second wash of 30 minutes was then performed.

Antibody, specific for PKCγ (epitope amino acids 684-697)(RBI, Sigma, Poole, Dorset) was diluted, in blocking solution, to a concentration of 1:1000 and the membrane was placed in this for three hours, with gentle agitation. After incubation the membrane was washed three times in blocking solution, ten minutes each wash. HRP-labelled secondary antibody, specific for rabbit (Amersham, Buckinghamshire), diluted to 1:1000 in blocking solution, was then poured over the membrane and incubation allowed to proceed for 1 hour, with gentle agitation. The membrane was finally washed three times in PBS-T, for ten minutes each wash.

Detection reagent was made up using an equal volume of ECL reagent 1 and 2 (Amersham). The membrane was incubated in this for 1 minute, then wrapped in Saran Wrap (Dow, Germany) and exposed to X-ray film (Konica, Tokyo, Japan) for five minutes before being developed using an X2 processor (Genetic Research Instrumentation, Dunmore, Essex).

The Western blot shown inFigure 11 demonstrates that full-length PKCγ is not expressed in the brain of the NNG3 rat. However, it is not possible to determine whether a truncated form of the protein is expressed, or whether PKCγ is missing in its entirety, from the brain of the NNG3 rat.

### Experiment 9 - Microinjection of PKCγ to allow expression in the brain

This work follows a protocol originally reported in Martes *et.al.* (Martres, M-P., Demeneix, B., Hanoun, N., Hamon, M. And Giros, B. (1998). Up-and down-expression of the dopamine transporter by plasmid DNA transfer in the rat brain. European Journal of Neurosciences, **10**, 3607-3616). The full length AS cDNA isolated, as described previously, was removed from the TOPO vector by restriction digest using HindIII and XbaI and cloned into the pRc/CMV2 vector (Invitrogen). DNA was then isolated from this clone using the EndoFree Plasmid Giga kit (Qiagen, Crawley, West Sussex), according to the manufactures protocol.

3 charge equivalents (taking into account that 1 *µ*g of DNA and 1 *µ*l of 0.1 M PEI correspond, respectively, to 3 nmoles of phosphate and 100 nmoles of amine nitrogen) of polyethylenimine (PEI) 25 kDa, (Aldrich, Poole, Dorset) were combined with 1*µ*g of DNA and vortexed for 30 seconds prior to injection.

Each animal was anaesthetised using Vetalar (ketamine hydrochloride; 100mg/ml; Code VM 14851/4009 Pharmacia & Upjohn Ltd, Animal Health Division, Crawley RH10 2LZ) and Rompun (Xylazine hydrochloride 2%; Bayer plc, Animal Health Business Group, Eastern Way, Bury St. Edmunds, Suffolk IP32 7AH) in a ratio of 2:1. Injections were given intraperitoneally at 1.1ml/kg.

Deep anaesthesia was established after 5-10 minutes using the 'pad pinching' technique where no reflexual withdrawing of the foot was observed when full anaesthesia was established. A small area on the head was then shaved using an electric razor.

The animal was placed into ear bars on a stereotaxic frame (Kopf, Munich, Germany). The head was held in_place using teeth bars and horizontally using a nose bar.

An incision was made using a scalpel blade and the skin was held back from the skull using skin retractors (Merck Ltd, Hunter Boulevard, Magna Park, Lutterworth, Leicestershire LE17 4XN). The skull was scraped using a scalpel blade to reveal Bregma and the area was cleaned and dried using a cotton bud. Bregma was marked using a pencil and confirmed independently prior to taking the Bregma co-ordinates. Lateral and anterior posterior co-ordinates were taken using the stereotaxic frame. The ventral co-ordinate was not taken at this point.

Co-ordinates were calculated, using the Paxinos and Watson stereotaxic brain atlas, and microinjection made at anterior/posterior (-3.8mm), lateral (+1.8mm) and ventral (-2.0mm) when compared with the co-ordinates of Bregma.

A 10*µ*l Hamilton microsyringe needle containing 2*µ*l of either pRc/CMV PKCγDNA in PEI, empty pRc/CMV vector in PEI or saline was placed directly over the brain area corresponding to these new co-ordinates. This was again marked using a pencil and confirmed independently.

The tip of a drill head (1mm) was placed over the pencil mark and a hole was drilled into the skull. The area was again dried using a cotton bud. The microsyringe needle was then placed over the hole and lowered into it slowly. The needle was then lowered into the brain to the appropriate depth according to the calculated ventral co-ordinate.

The needle was left in place for 2 minutes then 1µl solution was injected. The needle was left in place for a further minute. At this time another 1µl of solution was injected. The needle was left in place for a further 2 minutes before being removed slowly.

The hole was then sealed with dental cement (Redifast 250ml Code 002363, Wright Health group Ltd, Kingsway West Dundee), the animal was removed carefully from the stereotaxic frame and the incision was closed using standard cotton sutures.

The animal was then given a subcutaneous injection of 0.1ml Antisedan (atipamezole hydrochloride; 5mg/ml; Code VM 0057/4111, Pfizer Ltd; Ramsgate Road, Sandwich, Kent CT13 9LU) and allowed to recover before being placed back into its cage.

The experiment was repeated with microinjection at co-ordinates of anterior/posterior (-3.8mm), lateral (+1.8mm) and ventral (-3.5mm) when compared with the co-ordinates of Bregma.

### Experiment 10 - Animal behaviour analysis

Each animal was assessed 24hrs after surgery to establish that no adverse effects had resulted from the microinjection described above. Non-invasive locomotor tests were carried out after a further 24 hrs and repeated every 48hrs until day seven. Three series of tests were carried out on each animal prior to decapitation on the seventh day.

The test chosen was the inclined ramp test.

### Inclined ramp test

Six planks of wood were used ranging between 10-120mm. The six planks used were 10, 20, 50, 70, 90 and 120mm. Each rat was placed onto each plank assessed for its ability to maintain it's balance and walk down to the bottom of the plank without falling off. This was carried out once. If the rat could do this, it received a score of 1 and if it fell off the plank it received a score of 0. This was repeated for all planks and was video-recorded.

These two experiments demonstrate that the introduction of wild type PKCγ into the brain of NNG3 rats allows them to perform the inclined ramp test better than their uninjected counterparts. Preliminary evidence therefore suggests that PKCγ expression may help to rectify the Parkinsonian phenotype.

## Claims

1. Use of a polynucleotide fragment comprising the PKCγ gene encoding the type 1 subtype of protein kinase C in the manufacture of a medicament for treating a neurodegenerative disorder.

2. Use of a polynucleotide fragment according to claim 1 wherein the medicament is used to treat mammals.

3. Use of a polynucleotide fragment according to claim 1 wherein the medicament is used to treat humans.

4. Use of a polynucleotide fragment according to any preceding claim wherein the degenerative disorder is a degenerative disorder of the central nervous system.

5. Use of a polynucleotide fragment according to claim 4 wherein the degenerative disorder of the central nervous system is Alzheimer's Disease.

6. Use of a polynucleotide fragment according to claim 4 wherein the degenerative disorder of the central nervous system is associated with dopaminergic cell degeneration.

7. Use of a polynucleotide fragment according to claim 4 wherein the degenerative disorder of the central nervous system is a neurodegenerative disorder associated with movement impairment.

8. Use of a polynucleotide fragment according to either of claims 6 or 7 wherein the neurodegenerative disorder is selected from the group comprising Parkinson's Disease, Huntington's Disease/Chorea, Dementia with Lewy bodies, Multiple-system atrophy, Progressive supranuclear palsy, cortical-basal ganglionic (corticobasal) degeneration, vascular Parkinsonism and ballism.

9. Use of a polypeptide comprising protein kinase C type 1 in the manufacture of a medicament for treating a neurodegenerative disorder.

10. Use of a polypeptide according to claim 9 wherein the medicament is used to treat mammals.

11. Use of a polypeptide according to claim 9 wherein the medicament is used to treat humans.

12. Use of a polypeptide according to any of claims 9 to 11 wherein a degenerative disorder is a degenerative disorder of the central nervous system.

13. Use of a polypeptide according to claim 12 wherein the degenerative disorder of the central nervous system is Alzheimer's Disease.

14. Use of a polypeptide according to claim 12 wherein the degenerative disorder of the central nervous system is associated with dopaminergic cell degeneration.

15. Use of a polypeptide according to claim 12 wherein the degenerative disorder of the central nervous system is a neurodegenerative disorder associated with movement impairment.

16. Use of a polypeptide according to either of claims 14 or 15 wherein the neurodegenerative disorder is selected from the group comprising Parkinson's Disease, Huntington's Disease/Chorea, Dementia with Lewy bodies, Multiple-system atrophy, Progressive supranuclear palsy, cortical-basal ganglionic (corticobasal) degeneration, vascular Parkinsonism and ballism.

17. Use of a polypeptide according to any of claims 9 to 16 wherein the polypeptide is synthetic.

18. A method of testing an animal thought to have a neurodegenerative disorder comprising detecting the presence of mutation in the PKCγ gene or its associated promoter.

19. A method of testing an animal thought to be predisposed to having a neurodegenerative disorder comprising detecting the presence of mutation in the PCKγ gene or its associated promoter.

20. A method according to either of claims 18 or 19 wherein the animal is a mammal.

21. A method according to claim 20 wherein the mammal is a human.

22. A method according to either of claims 18 and 19 wherein the neurodegenerative disorder is a degenerative disorder of the central nervous system.

23. A method according to claim 22 wherein the degenerative disorder of the central nervous system is Alzheimer's Disease.

24. A method according to claim 22 wherein the degenerative disorder of the central nervous system is associated with dopaminergic cell degeneration.

25. A method according to claim 22 wherein the degenerative disorder of the central nervous systems is a neurodegenerative disorder associated with movement impairments.

26. A method according to either of claims 24 or 25 wherein the neurodegenerative disorder is selected from the group comprising Parkinson's Disease, Huntington's Disease/Chorea, Dementia with Lewy bodies, Multiple-system atrophy, Progressive supranuclear palsy, cortical-basal ganglionic (corticobasal) degeneration, vascular Parkinsonism and ballism.

27. A method according to either of claims 18 or 19 wherein the mutation results in a truncated product from the PKCγ gene being produced.

28. A method according to claim 27 wherein the mutation occurs in the 5' half of the gene.

29. A method according to claim 28 wherein the mutation is a point mutation at position 841 of the rat PKCγ gene or a similar region of the PKCγ gene from another species.

30. A method according to either of claims 18 or 19 wherein detection of the presence of the mutation in the PKCγ gene is achieved by detecting altered levels of the mRNA transcripts or mRNA precursor.

31. A method according to either of claims 18 or 19 wherein the mutation in the PKCγ gene is detected using antibodies raised to the truncated PKC type I polypeptide.

32. Use of a truncated PKCγ polynucleotide fragment for promoting nervous system degeneration for the production of animal models.

33. Use of a truncated PKC type I polypeptide for promoting nervous system degeneration for the production of animal models.

34. Use of PKCγ polynucleotide fragment encoding the PKC type I polypeptide in the manufacture of a medicament for preventing, delaying, treating or inhibiting degeneration of nervous system.

35. Use of a PKC type I polypeptide in the manufacture of a medicament for preventing, delaying, treating or inhibiting degeneration of nervous system.

36. A polynucleotide fragment encoding the PKC type I polypeptide for use in gene therapy.

37. A humanised monoclonal antibody specific for an epitope(s) located on a truncated polypeptide produced from the PKCγ gene.

38. An antibody according to claim 37 wherein the epitope(s) is/are located in the C terminal half of the PKC type I polypeptide.

39. An antibody according to claim 38 wherein the C terminal half of the polypeptide begins at amino acid number 282 and ends at the C terminus of the native polypeptide.

40. An antibody according to any of claims 37 to 39 wherein the antibody is a monoclonal antibody.

41. Use of an antibody according to claims 37 - 40 for the manufacture of a medicament for preventing, delaying, treating or inhibiting degeneration of the nervous system.

42. Use of an antibody according to claims 37 - 40 in a diagnostic assay for testing an human thought to have or be predisposed to having a neural degenerative disorder.

## Patentansprüche

1. verwendung eines Polynukleotidfragments umfassend das PKC_{Y}-Gen, welches für den Typ 1 Subtyp von proteinkinase C kodiert, bei der Herstellung eines Medikaments zur Behandlung einer neurodegenerativen Erkrankung.

2. Verwendung eines Polynukleotidfragments gemäß Anspruch 1, wobei das Medikament zur Behandlung von Säugetieren verwendet wird.

3. Verwendung eines Polynukleotidfragments gemäß Anspruch 1, wobei das Medikament zur Behandlung von Menschen verwendet wird.

4. Verwendung eines Polynukleotidfragments gemäß einem der vorangehenden Ansprüche, wobei die degenerative Erkrankung eine degenerative Erkrankung des zentralen Nervensystems ist.

5. Verwendung eines Polynukleotidfragments gemäß Anspruch 4, wobei die degenerative Erkrankung des zentralen Nervensystems Alzheimer'sche Krankheit ist.

6. Verwendung eines Polynukleotidfragments gemäß Anspruch 4, wobei die degenerative Erkrankung des zentralen Nervensystems mit Degeneration dopaminerger Zellen assoziiert ist.

7. Verwendung eines Polynukleotidfragments gemäß Anspruch 4, wobei die degenerative Erkrankung des zentralen Nervensystems eine mit Bewegungsbeeinträchtigung assoziierte neurodegenerative Erkrankung ist.

8. Verwendung eines Polynukleotidfragments gemäß einem der Ansprüche 6 oder 7, wobei die neurodegenerative Erkrankung ausgewählt ist aus der Gruppe umfassend Parkinson Krankheit, Huntington's Krankheit/Chorea, Dementia mit Lewy-Körpern, Multiple-System-Atropie, progressive supranukleare Lähmung, kortikal-basale ganglionische (kortikobasale) Degenerierung, vaskulärer Parkinsonismus und Ballismus.

9. Verwendung eines Polypeptids, welches Proteinkinase C von Typ 1 umfasst, bei der Herstellung eines Medikaments zur Behandlung einer neurodegenerativen Erkrankung.

10. Verwendung eines Polypeptids gemäß Ansprüchen 9 bis 11, wobei das Medikament zur Behandlung von Säugetieren verwendet wird.

11. Verwendung eines Polypeptids gemäß Anspruch 9, wobei das Medikament zur Behandlung von Menschen verwendet wird.

12. Verwendung eines Polypeptids gemäß einem der Ansprüche 9 bis 11, wobei eine degenerative Erkrankung eine degenerative Erkrankung des zentralen Nervensystems ist.

13. Verwendung eines Polypeptids gemäß Anspruch 12, wobei die degenerative Erkrankung des zentralen Nervensystems Alzheimer'sche Krankheit ist.

14. Verwendung eines Polypeptids gemäß Anspruch 12, wobei die degenerative Erkrankung des zentralen Nervensystems mit Degeneration dopaminerger Zellen assoziiert ist.

15. Verwendung eines Polypeptids gemäß Anspruch 12, wobei die degenerative Erkrankung des zentralen Nervensystems eine mit Bewegungsbeeinträchtigung assoziierte neurodegenerative Erkrankung ist.

16. Verwendung eines Polypeptids gemäß einem der Ansprüche 14 oder 15, wobei die neurodegenerative Erkrankung ausgewählt ist aus der Gruppe umfassend Parkinson Krankheit, Huntington's Krankheit/Chorea, Dementia mit Lewy-Körpern, Multiple-System-Atropie, progressive supranukleare Lähmung, kortikal-basale ganglionische (kortikobasale) Degenerierung, vaskulärer Parkinsonismus und Ballismus.

17. Verwendung eines Polypeptids gemäß einem der Ansprüche 9 bis 16, wobei das Polypeptid synthetisch ist.

18. verfahren zum Untersuchen eines Tieres, bei dem eine neurodegenerative Erkrankung vermutet wird, umfassend Detektieren der Gegenwart einer Mutation in dem PKCγ-Gen oder dem mit ihm assoziierten Promotor.

19. Verfahren zum Untersuchen eines Tieres, bei dem eine Prädisposition für eine neurodegenerative Erkrankung vermutet wird, umfassend Detektieren der Gegenwart einer Mutation in dem PKCγ-Gen oder dem mit ihm assoziierten Promotor.

20. Verfahren gemäß einem der Ansprüche 18 oder 19, wobei das Tier ein Säugetier ist.

21. Verfahren gemäß Anspruch 20, wobei das Säugetier ein Mensch ist.

22. Verfahren gemäß einem der Ansprüche 18 und 19, wobei die degenerative Erkrankung eine degenerative Erkrankung des zentralen Nervensystems ist.

23. Verfahren gemäß Anspruch 22, wobei die degenerative Erkrankung des zentralen Nervensystems Alzheimer'sche Krankheit ist.

24. Verfahren gemäß Anspruch 22, wobei die degenerative Erkrankung des zentralen Nervensystems mit Degeneration dopaminerger Zellen assoziiert ist.

25. Verfahren gemäß Anspruch 22, wobei die degenerative Erkrankung des zentralen Nervensystems eine mit Bewegungsneeinträchtigung assoziierte neurodegenerative Erkrankung ist.

26. Verfahren gemäß einem der Ansprüche 24 oder 25, wobei die neurodegenerative Erkrankung ausgewählt ist aus der Gruppe umfassend Parkinson Krankheit, Huntington's Krankheit/Chorea, Dementia mit Lewy-Körpern, Multiple-System-Atropie, progressive supranukleare Lähmung, kortikal-basale ganglionische (kortikobasale) Degenerierung, vaskulärer Parkinsonismus und Ballismus.

27. Verfahren gemäß einem der Ansprüche 18 oder 19, wobei die Mutation zu einem verkürzten Produkt des produziert werdenden PKCγ-Gens führt.

28. Verfahren gemäß Anspruch 27, wobei die Mutation in der 5'-Hälfte des Gens auftritt.

29. Verfahren gemäß Anspruch 28, wobei die Mutation eine Punktmutation an Position 841 des Ratten-PKC_{Y}-Gens oder einem ähnlichen Bereich des PKCγ-Gens einer anderen Spezies ist.

30. Verfahren gemäß einem der Ansprüche 18 oder 19, wobei Detektion der Gegenwart der Mutation im PKC_{Y}-Gen erreicht wird durch Detektion veränderter Gehalte des mRNA-Transkripts oder mRNA-Vorläufers.

31. Verfahren gemäß einem der Ansprüche 18 oder 19, wobei die Mutation im PKCy-Gen detektiert wird unter Verwendung von Antikörpern, welche gegen das verkürzte PKC-Typ I-Polypeptid erzeugt wurden.

32. Verwendung eines verkürzten PKC_{Y}-Polynukleotidfragments zum Fördern der Degenerierung des Nervensystems zur Produktion von Tiermodellen.

33. Verwendung eines verkürzten PKC-Typ I-Polypeptids zum Fördern der Degenerierung des Nervensystems zur Produktion von Tiermodellen.

34. Verwendung eines PKC_{Y}-Polynukleotidfragments, welches für das PKC-Typ 1-Polypeptid kodiert, bei der Herstellung eines Medikaments zu Prävention, Verzögerung, Behandlung oder Inhibierung der Degenerierung des Nervensystems.

35. Verwendung eines PKC-Typ 1-Polypeptids bei der Herstellung eines Medikaments zu Prävention, Verzögerung, Behandlung oder Inhibierung der Degenerierung des Nervensystems.

36. Polynukleotidfragment, welches für das PKC- Typ I-Polypeptid kodiert, zur Verwendung bei Gentherapie.

37. Humanisierter monoklonaler Antikörper, welcher spezifisch für ein Epitop/Epitope ist, welche/s auf einem verkürzten Polypeptid angeordnet ist/sind, welches von dem PKC_{Y}-Gen erzeugt wurde.

38. Antikörper gemäß Anspruch 37, wobei das Epitop/die Epitope in der C-terminalen Hälfte des PKC-Typ I-Polypeptids angeordnet ist/sind.

39. Antikörper gemäß Anspruch 38, wobei die C-terminale Hälfte des Polypeptids bei Aminosäure Nr. 282 beginnt und an dem C-Terminus des nativen Polypeptids endet.

40. Antikörper gemäß einem der Ansprüche 37 bis 39, wobei der Antikörper ein monoklonaler Antikörper ist.

41. Verwendung eines Antikörpers gemäß Ansprüchen 37 bis 40 für die Herstellung eines Medikaments zu Prävention, Verzögerung, Behandlung oder Inhibierung der Degenerierung des Nervensystems.

42. Verwendung eines Antikörpers gemäß Ansprüchen 37 bis 40 in einem diagnostischen Assay zur Untersuchung eines Menschen, von dem vermutet wird, dass er eine neurale degenerative Erkrankung hat oder für diese prädisponiert ist.

## Revendications

1. Utilisation d'un fragment de polynucléotide comprenant le gène PKCγ codant pour le sous-type de type 1 de la protéine kinase C dans la fabrication d'un médicament pour traiter une maladie neurodégénérative.

2. Utilisation d'un fragment de polynucléotide selon la revendication 1 dans laquelle le médicament est utilisé pour traiter des mammifères.

3. Utilisation d'un fragment de polynucléotide selon la revendication 1 dans laquelle le médicament est utilisé pour traiter des humains.

4. Utilisation d'un fragment de polynucléotide selon l'une quelconque des revendications précédentes dans laquelle la maladie dégénérative est une maladie dégénérative du système nerveux central.

5. Utilisation d'un fragment de polynucléotide selon la revendication 4 dans laquelle la maladie dégénérative du système nerveux central est la maladie d'Alzheimer.

6. Utilisation d'un fragment de polynucléotide selon la revendication 4 dans laquelle la maladie dégénérative du système nerveux central est associée à la dégénérescence des cellules dopaminergiques.

7. Utilisation d'un fragment de polynucléotide selon la revendication 4 dans laquelle la maladie dégénérative du système nerveux central est une maladie neurodégénérative associée à un trouble du mouvement.

8. Utilisation d'un fragment de polynucléotide selon l'une quelconque des revendications 6 ou 7 dans laquelle la maladie neurodégénérative est choisie dans le groupe comprenant la maladie de Parkinson, la maladie/chorée de Huntington, la démence à corps de Lewy, l'atrophie de systèmes multiples, la maladie de Steele-Richardson, la dégénérescence ganglionnaire corticale-basale (corticobasale), le syndrome parkinsonien vasculaire et le ballisme.

9. Utilisation d'un polypeptide comprenant la protéine kinase C de type 1 dans la fabrication d'un médicament pour traiter une maladie neurodégénérative.

10. Utilisation d'un polypeptide selon la revendication 9 dans laquelle le médicament est utilisé pour traiter des mammifères.

11. Utilisation d'un polypeptide selon la revendication 9 dans laquelle le médicament est utilisé pour traiter dés humains.

12. Utilisation d'un polypeptide selon l'une quelconque des revendications 9 à 11 dans laquelle la maladie dégénérative est une maladie dégénérative du système nerveux central.

13. Utilisation d'un polypeptide selon la revendication 12 dans laquelle la maladie dégénérative du système nerveux central est la maladie d'Alzheimer.

14. Utilisation d'un polypeptide selon la revendication 12 dans laquelle la maladie dégénérative du système nerveux central est associée à la dégénérescence des cellules dopaminergiques.

15. Utilisation d'un polypeptide selon la revendication 12 dans laquelle la maladie dégénérative du système nerveux central est une maladie neurodégénérative associée à un trouble du mouvement.

16. Utilisation d'un polypeptide selon l'une quelconque les revendications 14 ou 15 dans laquelle la maladie neurodégénérative est choisie dans le groupe comprenant la maladie de Parkinson, la maladie/chorée de Huntington, la démence à corps de Lewy, l'atrophie de systèmes multiples, la maladie de Steele-Richardson, la dégénérescence ganglionnaire corticale-basale (corticobasale), le syndrome parkinsonien vasculaire et le ballisme.

17. Utilisation d'un polypeptide selon l'une quelconque des revendications 9 à 16 dans laquelle le polypeptide est synthétique.

18. Procédé pour tester un animal suspecté d'avoir une maladie neurodégénérative comprenant la détection de la présence d'une mutation dans le gène PKCγ ou son promoteur associé.

19. Procédé pour tester un animal suspecté d'être prédisposé à avoir une maladie neurodégénérative comprenant la détection de la présence d'une mutation dans le gène PKCγ ou son promoteur associé.

20. Procédé selon l'une quelconque des revendications 18 ou 19 dans lequel l'animal est un mammifère.

21. Procédé selon la revendication 20 dans lequel le mammifère est un humain.

22. Procédé selon l'une quelconque des revendications 18 ou 19 dans lequel la maladie neurodégérative est une maladie dégénérative du système nerveux central.

23. Procédé selon la revendication 22 dans lequel la maladie dégénérative du système nerveux central est la maladie d'Alzheimer.

24. Procédé selon la revendication 22 dans lequel la maladie dégénérative du système nerveux central est associée à la dégénérescence des cellules dopaminergiques.

25. Procédé selon la revendication 22 dans lequel la maladie dégénérative du système nerveux central est une maladie neurodégénérative associée à un trouble du mouvement.

26. Procédé selon l'une quelconque des revendications 24 ou 25 dans lequel la maladie neurodégénérative est choisie dans le groupe comprenant la maladie de Parkinson, la maladie/chorée de Huntington, la démence à corps de Lewy, l'atrophie de systèmes multiples, là maladie de Steele-Richardson, la dégénérescence ganglionnaire corticale-basale (corticobasale), le syndrome parkinsonien vasculaire et le ballisme.

27. Procédé selon l'une quelconque des revendications 18 ou 19 dans lequel la mutation résulte en ce qu'un produit tronqué du gène PKCγ est généré.

28. Procédé selon la revendication 27 dans lequel la mutation se produit dans la moitié 5' du gène.

29. Procédé selon la revendication 28 dans lequel la mutation est une mutation ponctuelle à la position 841 du gène PKCγ de rat ou une région similaire du gène PKCγ d'une autre espèce.

30. Procédé selon l'une quelconque des revendications 18 ou 19 dans lequel la détection de la présence de la mutation dans le gène PKCγ est effectuée en détectant des taux altérés de produits de transcription d'ARNm ou de précurseur d'ARNm.

31. Procédé selon l'une quelconque des revendications 18 ou 19 dans lequel la mutation dans le gène PKCγ est détectée en utilisant des anticorps contre le polypeptide de PKC de type I tronqué.

32. Utilisation d'un fragment de polynucléotide PKCγ tronqué pour favoriser la dégénérescence du système nerveux pour la production de modèles animaux.

33. Utilisation d'un polypeptide de PKC de type I tronqué pour favoriser la dégénérescence du système nerveux pour la production de modèles animaux.

34. Utilisation d'un fragment de polynucléotide PKCγ codant pour le polypeptide de PKC de type I dans la fabrication d'un médicament pour prévenir, retarder, traiter ou inhiber la dégénérescence du système nerveux.

35. Utilisation d'un polypeptide de PKC de type I dans la fabrication d'un médicament pour prévenir, retarder, traiter ou inhiber la dégénérescence du système nerveux.

36. Fragment de polynucléotide codant pour le polypeptide de PKC de type I pour utilisation en thérapie génique.

37. Anticorps monoclonal humanisé spécifique d'un ou d'épitopes situés sur un polypeptide tronqué produit à partir du gène PKCγ.

38. Anticorps selon la revendication 37 dans lequel le ou les épitopes est/sont situés dans la moitié C-terminale du polypeptide de PKC de type I.

39. Anticorps selon la revendication 38 dans lequel la moitié C-terminale du polypeptide commence à l'acide aminé numéro 282 et termine au C terminal du polypeptide natif.

40. Anticorps selon l'une quelconque des revendications 37 à 39 dans lequel l'anticorps est un anticorps monoclonal.

41. Utilisation d'un anticorps selon les revendications 37 à 40 pour la fabrication d'un médicament pour prévenir, retarder, traiter ou inhiber la dégénérescence du système nerveux.

42. Utilisation d'un anticorps selon les revendications 37 à 40 dans un essai de diagnostic pour tester un humain suspecté d'avoir ou d'être prédisposé à avoir une maladie neurodégénérative.
